(19) Europäisches Patentamt
European Patent Office
Office européen des brevets

(11) EP 3 530 322 A1

(12) # EUROPÄISCHE PATENTANMELDUNG

(43) Veröffentlichungstag:
**28.08.2019 Patentblatt 2019/35**

(51) Int Cl.:
***A61Q 5/00*** *(2006.01)* ***A61K 8/87*** *(2006.01)*
***C08L 75/00*** *(2006.01)* ***C08L 79/00*** *(2006.01)*

(21) Anmeldenummer: **18158863.3**

(22) Anmeldetag: **27.02.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **KOSMETISCHE ZUSAMMENSETZUNG ZUR VERBESSERUNG DER BESTÄNDIGKEIT EINER FRISUR**

(57) Die vorliegende Erfindung betrifft eine Frisur-stabilisierende Zusammensetzung, aufweisend einen speziellen bevorzugt amorphen Polyurethanharnstoff mit Carboxy- oder Carboxylatgruppen. Weiterhin betrifft die Erfindung die Verwendung des speziellen bevorzugt amorphen Polyurethanharnstoff für die Herstellung einer Frisur-stabilisierenden Zusammensetzung sowie ein Verfahren zur Generierung einer wasserstabilen Frisur und ein Verfahren zur Herstellung der Frisur-stabilisierende Zusammensetzung.

EP 3 530 322 A1

## Beschreibung

Die vorliegende Erfindung betrifft eine Frisur-stabilisierende Zusammensetzung, aufweisend einen speziellen bevorzugt amorphen Polyurethanharnstoff mit Carboxy- oder Carboxylatgruppen. Weiterhin betrifft die Erfindung die Verwendung des speziellen bevorzugt amorphen Polyurethanharnstoff für die Herstellung einer Frisur-stabilisierenden Zusammensetzung sowie ein Verfahren zur Generierung einer wasserstabilen Frisur und ein Verfahren zur Herstellung der Frisur-stabilisierende Zusammensetzung.

Zur Gestaltung und Stabilisierung vielseitiger Frisuren werden Produkte eingesetzt, die als Haarfestiger bekannt sind. Haarfestiger gibt es meistens in Form von Schaumfestigern oder Haarsprays, wobei sie sich in ihrer Zusammensetzung kaum unterscheiden. Schaumfestiger werden auf das feuchte Haar als Hilfsmittel zur Modellierung der Frisur aufgetragen. Im Gegensatz hierzu, werden Haarsprays, Haarcremes, Haargele oder Haarwachse auf trockene fertig gestylte Haare zur Fixierung der Frisur für den Alltag an Luft aufgebracht.

In dem Falle von Haarsprays und Schaumfestigern liegen die Mittel zur Fixierung oder Gestaltung der Frisur üblicherweise als aus Aerosolbehältern, Quetschflaschen oder durch eine Pump-, Sprüh- oder Schäumvorrichtungen versprühbare Präparate vor, die aus einer alkoholischen, wässrigen oder wäßrig-alkoholischen Lösung von filmbildenden natürlichen oder synthetischen Polymeren bestehen. Diese Polymere können aus der Gruppe der nichtionischen, kationischen, amphoteren oder anionischen Polymeren ausgewählt werden.

Als filmbildende Polymere werden im Stand der Technik häufig anionische oder amphotere Polymere auf Basis von Acrylaten eingesetzt. Bekannt ist aber auch die Verwendung von Polyurethanen und Polyurethanharnstoffen als Filmbildner. So sind beispielsweise in der WO 2009/118105 A1 Haarfestiger Zusammensetzungen beschrieben, die einen Polyurethanharnstoff erhalten, der durch Umsetzung eines wasserunlöslichen nicht wasserdispergierbaren isocyanatfunktionellen Polyurethanprepolymers mit einer aminofunktionellen Verbindung erhältlich ist. Die hier offenbarten Haarfestiger Zusammensetzungen sind gut zur Stabilisierung von Frisuren bis zur nächsten Haarwäsche geeignet. Allerdings können hiermit keine Frisuren nachhaltig gestaltet werden, die eine Haarwäsche überdauern würden.

Aufgabe der vorliegenden Erfindung war es daher mindestens einen Nachteil des Standes der Technik zumindest zu einem Teil zu überwinden.

Weiterhin war es eine Aufgabe eine spezielle Zusammensetzung bereit zu stellen, die Frisurstabilisierend ist, insbesondere dazu geeignet ist bei Verwendung auf Haaren eine erhöhte Wasserbeständigkeit der hiermit geformten Frisur zu erzielen. Insbesondere war es eine Aufgabe eine Wasserbeständigkeit der mit der speziellen Zusammensetzung geformten Frisur über mehrere Waschgänge zu erzielen, insbesondere eine Wasser- und Waschbeständigkeit herbeizuführen, die ein erneutes Frisieren des Haares nach dem Wasserkontakt oder dem Waschen über mindestens einen, bevorzugt mehrere Wasserkontakt- oder Waschschritte unnötig macht.

Eine weitere Aufgabe war es ein Verfahren bereitzustellen, die es ermöglicht eine wasser- und/oder waschbeständige Frisur herzustellen, insbesondere eine Wasser- und Waschbeständigkeit herbeizuführen, die ein erneutes Frisieren des Haares nach dem Wasserkontakt oder dem Waschen über mindestens einen, bevorzugt mehrere Wasserkontakt- oder Waschschritte unnötig macht.

Es ist weiterhin eine Aufgabe der Erfindung, ein Verfahren zur Herstellung der speziellen Zusammensetzung mit den oben beschriebenen Eigenschaften bereitzustellen.

Eine Aufgabe der Erfindung ist es, eine Verwendung der speziellen Zusammensetzung zur Generierung von wasserfesten Frisuren bereitzustellen.

Mindestens eine der Aufgaben wird gelöst durch eine Zusammensetzung gemäß des Gegenstands des Anspruch 1 sowie dessen Verwendung zur Generierung von wasserfesten Frisuren. Besondere Ausführungsformen sind in den abhängigen Ansprüchen beschrieben. Wiederum ein Teil der Aufgaben wird durch die Ausführung des Verfahrens zur Herstellung des speziellen Polyurethanharnstoff gelöst.

[A1] Ein erster Gegenstand der Erfindung betrifft eine Frisur-stabilisierende Zusammensetzung, beinhaltend mindestens die folgenden Komponenten:

(V1) einen bevorzugt amorphen Polyurethanharnstoff (V1), welcher erhältlich ist, durch Umsetzung von wenigstens

A) einer bevorzugt aliphatischen Polyisocyanat-Komponente mit einer mittleren Isocyanatfunktionalität von $\geq 1{,}8$ und $\leq 2{,}6$,

B) einer polymeren Polyol-Komponente,

C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,

D) gegebenenfalls weiteren hydrophilierenden Komponenten, die unterschiedlich sind von C2),

E) gegebenenfalls hydroxyfunktionellen Verbindungen mit einem Molekulargewicht von 62 bis 399 mol/g,

F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und

G) gegebenenfalls einer aliphatischen Polyisocyanat-Komponente mit einer mittleren Isocyanatfunktionalität von $> 2,6$ und $\leq 4$,

wobei der Polyurethanharnstoff mindestens eine der Komponenten C2) oder D) aufweist, und wobei der Polyurethanharnstoff Carboxygruppen oder Carboxylatgruppen aufweist;

(V2) eine Polycarbodiimid-Komponente.

Unter einer Frisur-stabilisierenden Zusammensetzung wird eine Zusammensetzung verstanden, die es ermöglicht nach Aufbringen auf dem Haar eines Benutzers und eines anschließenden Formens des Haares zu einer Frisur, eine höhere Wasserbeständigkeit oder Waschbeständigkeit zu erzielen als beim Formen der Frisur ohne das Aufbringen der Zusammensetzung auf das Haar. Dabei kann die Frisur-stabilisierende Zusammensetzung als eine Komponente auf das Haar aufgebracht werden oder als zwei. Erfolgt das Behandeln des Haares durch Aufbringen der Frisur-stabilisierende Zusammensetzung in zwei Schritten, so wird zunächst das Polyurethanharnstoff (V1) als einzelne Komponente auf das Haar aufgebracht und in einem anschließenden Schritt die Polycarbodiimid-Komponente (V2) oder umgekehrt. Wird das Behandeln des Haares durch Aufbringen der Frisur-stabilisierende Zusammensetzung in einem Schritt durchgeführt, so liegen die beiden Komponenten (V1) und (V2) bereits vor dem Behandeln des Haares gemischt vor.
Unter Wasserbeständigkeit wird erfindungsgemäß verstanden, dass die Beständigkeit der mit der erfindungsgemäßen Zusammensetzung geformten Frisur gegenüber Wasser geprüft bzw. bestimmt wird. Hierbei wird das Haar in einer Spülbehandlung mindestens zu einem Teil mit flüssigem Wasser umgeben, also hierin getränkt oder gespült. Bei dem Wasser kann es sich um jede Form von flüssigem Wasser handeln, dass im Alltag mit dem Haar in Kontakt kommt. Beispiele hierfür sind Trinkwasser, Regenwasser, Schwimmbadwasser (gechlort, ozonisiert oder anderweitig keimfrei gehalten), Meerwasser oder destilliertes Wasser.
Unter Waschbeständigkeit wird erfindungsgemäß verstanden, dass die Beständigkeit der mit der erfindungsgemäßen Zusammensetzung geformten Frisur gegenüber tensidhaltigem Wasser geprüft bzw. bestimmt wird, wobei das Haar bei dieser Waschbehandlung komplett mit dem tensidhaltigen Wasser umgeben wird, also hierin gespült wird.
Bevorzugt ist das Formen des Haares zu einer Frisur ausgewählt aus der Gruppe bestehend aus:

a. Einem Glätten von lockigem Haar;
b. Einem Lockeneinbringen in glattes Haar;
c. Einem Verstärken von Locken in bereits gelocktem Haar;
d. Einer Kombination aus mindestens zwei von a. bis c..

Das Glätten von lockigem Haar unter a. kann dabei auf jede dem Fachmann bekannt Art und Weise, insbesondere mittels einer Glättvorrichtung erfolgen. Bevorzugt erfolgt das Glätten von lockigem Haar mittels eines Glätteisens, eines Glättstabes oder einer ähnlichen Glättvorrichtung, die hierfür geeignet ist. Bevorzugt ist die Glättvorrichtung so ausgestaltet, dass sie mit Hilfe von auf mindestens 150°C erhitzbaren Elementen ausgestattet ist. Bevorzugt ist die Glättvorrichtung dazu geeignet, Locken zu einem Glättungsgrad von mindestens 50%, oder bevorzugt zu mindestens 80%, oder bevorzugt zu mindestens 90% zu glätten. Die Ermittlung des Glättungsgrades erfolgt dabei durch Bestimmung der Breite einer Haarsträhne vor und nach dem Glätten mittels eines Lineals. Ein Glättungsgrad von 50%, bzw. 80%, bzw. 90% bedeutet, dass die Breite der Haarsträhne nach dem Schritt a. um 50%, bzw. 80%, bzw. 90% schmaler gegenüber der Breite vor dem Schritt a. geworden ist.
Das Lockeneinbringen in glattes Haar unter b. kann dabei auf jede dem Fachmann bekannt Art und Weise, insbesondere mittels einer Lockenerzeugungsvorrichtung erfolgen. Bevorzugt erfolgt das Lockigmachen von glattem Haar mittels eines Lockenstabes oder einer ähnlichen Lockenerzeugungsvorrichtung, die hierfür geeignet ist. Bevorzugt ist die Lockenerzeugungsvorrichtung so ausgestaltet, dass sie mit Hilfe von Locken erzeugenden Elementen, die bevorzugt leicht erwärmt werden können, beispielsweise auf Temperaturen von 40 bis 80 °C beinhaltet. Bevorzugt ist die Lockenerzeugungsvorrichtung dazu geeignet, Locken zu einem Lockungsgrad von mindestens 50%, oder bevorzugt zu mindestens 80%, oder bevorzugt zu mindestens 90% zu erzeugen. Die Ermittlung des Lockungsgrades erfolgt dabei durch Bestimmung der Länge einer Haarsträhne vor und nach dem Lockeneinbringen mittels eines Lineals. Ein Lockungsgrad von 50%, bzw. 80%, bzw. 90% bedeutet, dass die Länge der Haarsträhne nach dem Schritt b. um 50%, bzw. 80%, bzw. 90% kürzer ist als vor dem Schritt b..

Das Verstärken der Locken unter c. erfolgt bevorzugt auf die gleiche Weise, wie das Einbringen der Locken unter b. Alternativ oder zusätzlich kann das Verstärken der Locken unter c. durch die erfindungsgemäße Zusammensetzung in bevorzugt feuchtes Haar erfolgen, wobei das Haar anschließend mit oder ohne Hilfsmittel getrocknet wird. Bevorzugt ist die Lockenerzeugungsvorrichtung dazu geeignet, Locken zu einem Lockungsgrad von mindestens 30%, oder bevorzugt zu mindestens 40%, oder bevorzugt zu 50% zu erzeugen. Die Ermittlung des Lockungsgrades erfolgt dabei durch Bestimmung der Länge einer Haarsträhne vor und nach dem Lockeneinbringen mittels eines Lineals. Ein Lockungsgrad von 30%, bzw. 40%, bzw. 50% bedeutet, dass die Länge der Haarsträhne nach dem Schritt b. um 30%, bzw. 40%, bzw. 50% kürzer ist als vor dem Schritt c..

Bevorzugt bleibt die Form des frisierten Haares mindestens zu 5 %, bevorzugt zu mindestens 7%, oder bevorzugt zu mindestens 10 % nach Wasserkontakt erhalten. Dabei kann der Grad der Erhaltung der Form des frisierten Haares bevorzugt stark in Abhängigkeit von der Art der Behandlung variieren. So macht es einen Unterschied, ob die Haare vor der erfindungsgemäßen Verwendung der erfindungsgemäßen Zusammensetzung geglättet wurden oder ob die Haare gelockt wurden. Der Grad der Erhaltung der Form des frisierten Haares wird im Folgenden auch als "styling retention" bezeichnet.

Bevorzugt bleibt bei der Stabilisierung der Frisur durch die erfindungsgemäße Zusammensetzung mittels einer Glättung des Haares unter a. mindestens zu 50%, oder bevorzugt mindestens zu 60 %, oder bevorzugt mindestens zu mindestens 70 % oder zu mindestens 80 %, oder zu mindestens 90 % nach Wasserkontakt erhalten oder die Lockung des Haares unter b. zu mindestens 5%, oder bevorzugt zu mindestens 7 %, oder bevorzugt zu mindestens 10 % oder die Lockung unter c. zu mindestens 5%, oder bevorzugt zu mindestens 7 %, oder bevorzugt zu mindestens 10 % erhalten.

Bevorzugt weist die Frisur-stabilisierende Zusammensetzung ein Gewichts-Verhältnis des Polyurethanharnstoff (V1) zu der Polycarbodiimid-Komponente (V2) in einem Bereich von 10:1 bis 1:2 oder bevorzugt in einem Bereich von 5:1 bis 1:1,5, oder bevorzugt in einem Bereich von 4:1 bis 1:1 auf.

Bevorzugt weist die Reaktionsmischung aus (V1) und (V2) bei der Herstellung der Frisur-stabilisierende Zusammensetzung ein molares Verhältnis der Carboxy- oder Carboxylatgruppen aus (V1) zu Carbodiimidgruppen aus (V2) in einem Bereich von 5:1 bis 1:5, oder bevorzugt in einem Bereich von 2:1 bis 1:2 oder bevorzugt in einem Bereich von 1,3:1 bis 1: 1,3 auf.

Bevorzugt ist der Polyurethanharnstoff (V1) der Zusammensetzung amorph. Amorph bedeutet im Sinne dieser Erfindung, dass der Polyurethanharnstoff im in der im folgenden ausgeführten Messmethode genannten Temperaturbereich keine oder nur so geringe kristalline Anteile ausbildet, dass mittels der beschriebenen DSC Messungen nur ein oder mehrere Glasübergangspunkte $T_g$, aber keine Schmelzbereiche mit einer Schmelzenthalpie ≥ 20 J/g in dem genannten Temperaturbereich gefunden werden können.

Bevorzugt weist die Frisur-stabilisierende Zusammensetzung ein Gewichts-Verhältnis des Polyurethanharnstoff (V1) zu der Polycarbodiimid-Komponente (V2) in einem Bereich von 80:1 bis 1,2:1 oder bevorzugt in einem Bereich von 60:1 bis 2:1, oder bevorzugt in einem Bereich von 40:1 bis 4:1 auf.

Bevorzugt amorphe Polyurethanharnstoffe im Sinne der Erfindung sind polymere Verbindungen, die mindestens zwei, bevorzugt mindestens drei Urethan-Gruppen-haltige Wiederholungseinheiten aufweisen:

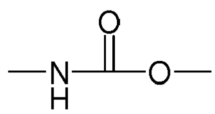

.

Erfindungsgemäß weisen die bevorzugt amorphen Polyurethanharnstoffe herstellungsbedingt auch Harnstoff-Gruppen-haltige Wiederholungseinheiten auf,

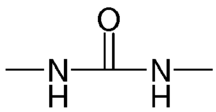

wie sie insbesondere bei der Umsetzung von isocyanatterminierten Präpolymeren mit aminofunktionellen Verbindungen gebildet werden.
Unter ionogenen Gruppen werden im Sinne dieser Erfindung solche funktionellen Gruppen verstanden, die in der Lage sind, beispielsweise durch Neutralisation mit einer Base, ionische Gruppen zu bilden.
Der Polyurethanharnstoff (V1) weist erfindungsgemäß Carboxygruppen oder Carboxylatgruppen auf. Diese können durch jede der verwendeten Komponenten A) bis G) oder weiteren verwendeten Komponenten in den Polyurethanharnstoff eingebracht werden. Bevorzugt werden die Carboxygruppen oder Carboxylatgruppen durch die Komponente B) eingebracht.
Bevorzugt weist der Polyurethanharnstoff (V1) der Zusammensetzung einen Gehalt an Carboxygruppen und/oder Carboxylatgruppen in einem Bereich von 0,01 bis 10 Gew.-%, oder bevorzugt in einem Bereich von 0,02 bis 8 Gew.-%, oder bevorzugt in einem Bereich von 0,05 bis 5 Gew.-%, bezogen auf die Gesamtmasse des Polyurethanharnstoffs auf.
Beispiele für Komponenten, die Carboxygruppen bzw. Carboxylatgruppen enthalten und als Bausteine für die Herstellung von (V1) benutzt werden können sind Dimethylolpropionsäure, Hydroxypivalinsäure, natürliche sowie nicht-natürliche Aminosäuren, wie 6-Aminohexansäure, Alanin, Asparaginsäure, Glutaminsäure, Glutamin, Glycin, Lysin, Leucin, Isoleucin oder Mischungen aus mindestens zwei hiervon.
Die Komponente A) kann jedes Polyisocyanat sein, das der Fachmann hierfür verwenden würde. Als Komponente A) bevorzugt geeignete Polyisocyanate sind insbesondere die dem Fachmann an sich bekannten, bevorzugt aliphatischen Polyisocyanate mit einer mittleren Isocyanatfunktionalität von ≥ 1,8 und ≤ 2,6. Der Begriff aliphatisch umfasst dabei auch cycloaliphatische und/oder araliphatische Polyisocyanate.
Unter der mittleren Isocyanatfunktionalität wird dabei die durchschnittliche Anzahl an Isocyanatgruppen pro Molekül verstanden.

[A2]Bevorzugte Polyisocyanate sind solche des Molekulargewichtsbereichs von 140 bis 336 g/mol. In einer bevorzugten Ausführungsform der Frisur-stabilisierenden Zusammensetzung ist das Polyisocynat der Polyisocyanatkomponent A) ausgewählt aus der Gruppe bestehend aus Toluol-2,4-diisocyanat (TDI), 2,2'-Diphenylmethandiisocyanat (2,2'-MDI), 2,4'-Diphenylmethandiisocyanat (4,4'-MDI), 4,4'-Diphenylmethandiisocyanat (4,4'-MDI), 4-Di¬iso-¬cyanato-butan (BDI), 1,5-Pentandiisocyanat, (PDI) 1,6-Di¬isocyanato¬hexan (HDI), 1,3-Bis(isocyanatomethyl)benzol (1,3-Xylylendiisocyanat, XDI), 1,4-Bis(isocyanatomethyl)benzol (1,4-Xylylendiisocyanat, XDI), 1,3-Bis(1-isocyanato-1-methyl-ethyl)benzol (TMXDI), 1,4-Bis(1-isocyanato-1-methyl-ethyl)benzol (TMXDI), 4-Isocyanatomethyl-1,8-octandiisocyanat (Trisisocyanatono¬inan (TIN)), 2-Methyl-1,5-diisocyanatopentan, 1,5-Di¬isocyanato-2,2-dimethyl-pentan, 2,2,4- bzw. 2,4,4-Trimethyl-1,6-diisocyanato¬hexan, 1,10-Di¬iso¬cyanato¬decan sowie die cycloaliphatischen Diisocyanate 1,3-bzw. 1,4-Di¬isocyanatocyclohexan, 1,4-Di¬isocyanato-3,3,5-trimethylcyclohexan, 1,3-Diisocyanato-2(4)-methylcyclohexan, 1-Iso¬cyanato-3,3,5-tri-imethyl-5-isocyanatomethylcyclohexan (Isopho¬ron¬diisocya¬nat, IPDI), 1-Isocyanato-1-me¬thyl-4(3)isocyanatome¬thylcyclo¬hexan, 1,8-Diisocya¬nato-p-menthan, 4,4'-Diisocyanato-1,1'-bi(cyclohexyl), 4,4'-Diisocyanato-3,3'-dimethyl-1,1'-bi(cyclohexyl), 4,4'-Diisocyanato-2,2',5,5'-tetramethyl-1,1'-bi(cyclohexyl), 4,4' und/oder 2,4'-Diisocyanato¬dicyclohexylme¬than, 4,4' Diisocyanato-3,3'-dimethyldicyclohexylmethan, 4,4' Diisocyanato-3,3',5,5'-tetra¬methyl-dicyclohexylmethan, 1,3-Diisocyanatoadamantan, und 1,3-Dimethyl-5,7-diisocyana-toadamantan oder beliebige Gemi¬sche solcher Isocyanate. Ganz besonders bevorzugt sind die Polyisocyanate ausgewählt aus 1,4-Butylendiisocyanat, 1,5-Pentamethy¬lendiisocyanat (PDI), 1,6-Hexamethy¬lendiisocyanat (HDI), Isophorondiisocyanat (IPDI), 2,2,4- und/oder 2,4,4-Trimethyl¬hexa¬methy¬len¬¬diiso¬cyanat, die isomeren Bis-(4,4'-isocyanatocyclo¬hexyl)¬methane oder deren Mischungen beliebigen Isomeren¬gehalts (H12-MDI), 1,4-Cyclohexylendi¬isocyanat, 4-Isocyanatomethyl-1,8-octandiisocyanat (Nonantriisocyanat) sowie Alkyl-2,6-diisocyanatohexanoate (Lysin¬diisocyanate) mit C1-C8-Alkylgruppen oder Mischungen aus mindestens zwei hiervon.
Neben den vorstehend genannten Polyisocyanaten können auch modifizierte Diisocyanate, die eine mittlere Isocyanatfunktionalität ≥ 2 und ≤ 2,6 aufweisen, mit Uretdion-, Isocyanurat-, Urethan-, Allophanat-, Biuret-, Iminooxadiazindion- oder Oxadiazintrionstruktur sowie Mischungen aus diesen und/oder den oben stehenden anteilig eingesetzt werden.
Bevorzugt handelt es sich um Polyisocyanate oder Polyisocyanatgemische der vorstehend genannten Art mit ausschließlich aliphatisch oder cycloaliphatisch gebundenen Isocyanatgruppen oder Mischungen aus diesen und einer mittleren NCO-Funktionalität der Mischung von ≥ 1,8 und ≤ 2,6 und besonders bevorzugt ≥ 2,0 und ≤ 2,4.

[A3] In einer bevorzugten Ausführungsform der Frisur-stabilisierenden Zusammensetzung enthält die Komponente A) ein aliphatisches oder cycloaliphatisches Polyisocyanat. Bevorzugt ist das Polyisocyanat der Polyisocyanat-Komponente A) ausgewählt aus der Gruppe bestehend aus HDI, IPDI und/oder H12-MDI oder deren Modifikationsprodukten, ganz besonders bevorzugt ausgewählt aus HDI und/oder IPDI.
In einer insbesondere bevorzugten Variante liegen als Komponente A) IPDI und HDI im Gemisch vor.

Bevorzugt liegt das Gewichtsverhältnis von IPDI:HDI für die Polyisocyanat-Komponente A) in einem Bereich von 1,05 bis 10, besonders bevorzugt in einem Bereich von 1,1 bis 5, und ganz besonders bevorzugt in einem Bereich von 1,1 bis 1,5.

In einer bevorzugten Ausführungsform wird zur Herstellung des erfindungsgemäß verwendeten bevorzugt amorphen Polyurethanharnstoffs ≥ 5 und ≤ 40 Gew.-% der Komponente A) und besonders bevorzugt ≥ 10 und ≤ 35 Gew.-% der Komponente A), jeweils bezogen auf die Gesamtmasse des bevorzugt amorphen Polyurethanharnstoffs, eingesetzt.

Weiterhin bevorzugt wird zur Herstellung des bevorzugt amorphen Polyurethanharnstoffs auch die Komponente G), eine aliphatische Polyisocyanat-Komponente mit einer mittleren Isocyanatfunktionalität (mittlere Anzahl Isocyanatgruppen pro Molekül) von > 2,6 und ≤ 4, vorzugsweise ≥ 2,8 und ≤ 3,8 eingesetzt. Die Komponente G) wird dabei bevorzugt im Gemisch mit Komponente A) eingesetzt.

Als Komponente G) besonders geeignet sind oligomere Diisocyanate, die eine Funktionalität > 2,6 und ≤ 4, vorzugsweise ≥ 2,8 und ≤ 3,8 aufweisen, mit Isocyanurat-, Urethan-, Allophanat-, Biuret-, Iminooxadiazindion- oder Oxadiazintrionstruktur. Ganz besonders bevorzugt enthält G) Isocyanuratstrukturen.

Bevorzugt besteht die aliphatische Polyisocyanat-Komponente G) aus einem aliphatischen oder cycloaliphatischen Polyisocyanat-Oligomer basierend auf HDI, IPDI und/oder H12-MDI, ganz besonders bevorzugt basierend auf HDI.

Das Molverhältnis der NCO-Gruppen aus Komponente A) zu Komponente G) beträgt dabei bevorzugt 100:0,5 bis 100: 50; besonders bevorzugt 100: 2 bis 100: 15 und ganz besonders bevorzugt 100:3 bis 100: 8.

Bevorzugt wird zur Herstellung des erfindungsgemäß verwendeten bevorzugt amorphen Polyurethanharnstoffs ≥ 0 und ≤ 10 Gew.-% der Komponente G) und besonders bevorzugt ≥ 0,1 und ≤ 3 Gew.-% der Komponente G, jeweils bezogen auf die Gesamtmasse des bevorzugt amorphen Polyurethanharnstoffs, eingesetzt.

Geeignete di- oder höherfunktionelle polymere Polyole B) sind Verbindungen mit mindestens zwei gegenüber Isocyanaten reaktionsfähigen Wasserstoffatomen und einem mittleren Molekulargewicht bevorzugt einem Bereich von 400 bis 10000 g/mol, oder bevorzugt von 500 bis 8000 g/mol, oder bevorzugt von 1000 bis 5000 g/mol, bestimmt durch Gelpermeationschromatographie gegenüber Polystyrol-Standard in Tetrahydrofuran bei 23°C. Beispiele für geeignete Aufbaukomponenten sind Polyether, Polyester, Polycarbonate, Polylactone und Polyamide. Bevorzugte Polyole B) weisen 2 bis 4, besonders bevorzugt 2 bis 3 Hydroxylgruppen auf. Auch Gemische verschiedener derartiger Verbindungen kommen in Frage.

Als Polyesterpolyole kommen insbesondere lineare Polyesterdiole oder auch schwach verzweigte Polyesterpolyole in Betracht, wie sie in bekannter Weise aus aliphatischen, cycloaliphatischen oder aromatischen Di- oder Polycarbonsäuren, wie z.B. Bernstein-, Methylbernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal-, Cyclohexandicarbon-, Malein-, Fumar-, Malon- oder Trimellitsäure sowie Säureanhydride, wie o-Phthal-, Trimellit- oder Bernsteinsäureanhydrid oder deren Gemische mit mehrwertigen Alkoholen, wie z.B. Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12 oder deren Gemische, gegebenenfalls unter Mitverwendung höherfunktioneller Polyole, wie Trimethylolpropan, Glycerin oder Pentaerythrit, hergestellt werden können. Als mehrwertige Alkohole zur Herstellung der Polyesterpolyole kommen natürlich auch cycloaliphatische und/oder aromatische Di- und Polyhydroxylverbindungen in Frage. Anstelle der freien Polycarbonsäure können auch die entsprechenden Polycarbonsäureanhydride oder entsprechende Polycarbonsäureester von niedrigen Alkoholen oder deren Gemische zur Herstellung der Polyester verwendet werden.

Selbstverständlich kann es sich bei den Polyesterpolyolen auch um Homo- oder Mischpolymerisate von Lactonen handeln, die bevorzugt durch Anlagerung von Lactonen oder Lactongemischen, wie Butyrolacton, ε-Caprolacton und/oder Methyl-ε-caprolacton an die geeignete di- und/oder höherfunktionelle Startermoleküle, wie z.B. die vorstehend als Aufbaukomponenten für Polyesterpolyole genannten niedermolekularen, mehrwertigen Alkohole erhalten werden. Die entsprechenden Polymerisate des ε-Caprolactons sind bevorzugt.

Besonders bevorzugt sind weitgehend lineare Polyesterpolyole, die als Aufbaukomponenten Adipinsäure und Butandiol-1,4 und/oder Hexandiol-1,6 und/oder 2,2-Dimethyl-1,3-propandiol enthalten.

Auch Hydroxylgruppen aufweisende Polycarbonate kommen als Polyhydroxylkomponenten in Betracht, z.B. solche, die durch Umsetzung von Diolen wie 1,4-Butandiol und/oder 1,6-Hexandiol mit Diarylcarbonaten, wie z.B. Diphenylcarbonat, Dialkylcarbonaten, wie z. B. Dimethylcarbonat oder Phosgen hergestellt werden können. Durch die zumindest teilweise Verwendung von Hydroxylgruppen aufweisenden Polycarbonaten kann die Hydrolysebeständigkeit der Polyurethan- bzw. Polyurethan-Harnstoff-Dispersionen verbessert werden.

Bevorzugt sind Polycarbonate, die durch Umsetzung von 1,6-Hexandiol mit Dimethylcarbonat hergestellt wurden.

Als Polyetherpolyole geeignet sind z.B. die Polyadditionsprodukte der Styroloxide, des Ethylenoxids, Propylenoxids, Tetrahydrofurans, Butylenoxids, Epichlorhydrins, sowie ihre Mischadditions- und Pfropfprodukte, sowie die durch Kondensation von mehrwertigen Alkoholen oder Mischungen derselben und die durch Alkoxylierung von mehrwertigen Alkoholen, Aminen und Aminoalkoholen gewonnenen Polyetherpolyole. Als Polyol-Komponenten B) geeignete Polyetherpolyole sind die Homo-, Misch- und Pfropfpolymerisate des Propylenoxids und des Ethylenoxids, welche durch Anlagerung der genannten Epoxide an niedermolekulare Di- oder Triole, wie sie oben als Aufbaukomponenten für Polyesterpolyole genannte werden oder an höherfunktionelle niedermolekulare Polyole wie z.B. Pentaerythrid oder Zucker oder an Wasser zugänglich sind.
Besonders bevorzugte di- oder höherfunktionelle Polyole B) sind Polyesterpolyole, Polylactone und Polycarbonate. Die bevorzugt als Komponente B) eingesetzten polymeren Polyether-Polyole weisen bevorzugt zahlenmittlere Molekulargewichte in einem Bereich von 400 bis 8000 g/mol, bevorzugt in einem Bereich von 600 bis 6000 g/mol, oder bevorzugt in einem Bereich von 1000 bis 3000 g/mol, bestimmt durch Gelpermeationschromatographie gegenüber Polystyrol-Standard in Tetrahydrofuran bei 23°C, und/oder einer OH-Funktionalität von bevorzugt in einem Bereich von 1,5 bis 6, oder bevorzugt in einem Bereich von 1,8 bis 3, oder bevorzugt in einem Bereich von 1,9 bis 2,1 auf. Der Ausdruck "polymere" Polyether-Polyole bedeutet hier insbesondere, dass die genannten Polyole mindestens zwei, bevorzugt mindestens drei miteinander verbundene Wiederholungseinheiten aufweisen.
Das zahlenmittlere Molekulargewicht wird im Rahmen dieser Anmeldung stets bestimmt durch Gelpermeationschromatographie (GPC) in Tetrahydrofuran bei 23°C. Es wird dabei vorgegangen nach DIN 55672-1: "Gelpermeationschromatographie, Teil 1 - Tetrahydrofuran als Elutionsmittel" (SECurity GPC-System von PSS Polymer Service, Flussrate 1,0 ml/min; Säulen: 2xPSS SDV linear M, 8×300 mm, 5 μm; RID-Detektor). Dabei werden Polystyrolproben bekannter Molmasse zur Kalibrierung verwendet. Die Berechnung des zahlenmittleren Molekulargewichts erfolgt softwaregestützt. Basislinienpunkte und Auswertegrenzen werden entsprechend der DIN 55672 Teil 1 festgelegt.
Geeignete Polyetherpolyole sind beispielsweise die an sich bekannten Additionsprodukte von Styroloxid, Ethylenoxid, Propylenoxid, Butylenoxid und/oder Epichlorhydrin an di- oder polyfunktionelle Startermoleküle. So sind insbesondere Polyalkylenglykole, wie Polyethylen-, Polypropylen- und/oder Polybutylenglykole anwendbar, insbesondere mit den oben genannten bevorzugten Molekulargewichten. Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol.

[A4] In einer bevorzugten Ausführungsform der Frisur-stabilisierenden Zusammensetzung enthält die Komponente B) Poly(propylenglykol)polyetherpolyole. Bevorzugt beinhaltet die Frisur-stabilisierende Zusammensetzung Poly(propylenglykol)polyetherpolyole in einem Bereich von 50 bis 100 Gew.-%, oder bevorzugt in einem Bereich von 70 bis 100 Gew.-% oder bevorzugt in einem Bereich von 90 bis 100 Gew.-%, besonders bevorzugt zu 100 Gew.-%, jeweils bezogen auf das Gesamtgewicht der Komponente B).
Weist die Komponente B) mehr als 50 Gew.-% Poly(propylenglykol)polyetherpolyole, bezogen auf die Summe aller Bestandteile der Komponente B) auf, so wird gegebenenfalls eine geringe Menge an Komponente F) eingesetzt oder bevorzugt Komponente F) gar nicht eingesetzt. Sollte die Komponente B) zur Herstellung der Frisur-stabilisierenden Zusammensetzung mehr als 10 Gew.-%, bevorzugt mehr als 20 Gew.-%, oder bevorzugt mehr als 30 Gew.-%, bezogen auf die Summe aller Bestandteile der Komponente B)Poly(tetramethylen)polyetherpolyole aufweisen, so ist es bevorzugt, dass Komponente F) anwesend ist.

[A5] In einer bevorzugten Ausführungsform der Frisur-stabilisierenden Zusammensetzung weist die Komponente B) eine mittlere Molekularmasse in einem Bereich von 400 bis 4000 g/mol, oder bevorzugt in einem Bereich von 500 bis 3500 g/mol, oder bevorzugt in einem Bereich von 800 bis 3000 g/mol auf.

[A6] In einer bevorzugten Ausführungsform der Zusammensetzung enthält die Komponente B) ein Gemisch aus Poly(propylenglykol)polyetherpolyole mit unterschiedlichem mittleren Molekulargewicht oder besteht daraus, wobei sich die Poly(propylenglykol)polyetherpolyole in ihren zahlenmittleren Molekulargewichten um wenigstens 100 g/mol, bevorzugt um wenigstens 200 g/mol, oder bevorzugt um wenigstens 400 g/mol, oder bevorzugt um wenigstens 800 g/mol, oder bevorzugt um wenigstens 1000 g/mol unterscheiden. Bevorzugt unterscheiden sich die zahlenmittleren Molekulargewichte der Poly(propylenglykol)polyetherpolyole um nicht mehr als 5000 g/mol, oder um nicht mehr als 4000 g/mol, oder um nicht mehr als 3000 g/mol.
Bevorzugt enthält die Komponente B) ein Gemisch aus Poly(propylenglykol)polyetherpolyolen I mit einem zahlenmittleren Molekulargewichte Mn von ≥ 400 und ≤ 1500 g/mol, besonders bevorzugt von ≥ 600 und ≤ 1200 g/mol, ganz besonders bevorzugt von 1000 g/mol und Poly(propylenglykol)polyetherpolyolen II mit einem zahlenmittleren Molekulargewichte Mn von ≥ 1500 und ≤ 8000 g/mol, besonders bevorzugt von ≥ 1800 und ≤ 3000 g/mol, ganz besonders bevorzugt von 2000 g/mol.
Das Gewichtsverhältnis der Poly(propylenglykol)polyetherpolyolen I zu den Poly(propylenglykol)polyetherpolyolen II liegt bevorzugt im Bereich von 0,01 bis 10, besonders bevorzugt im Bereich von 0,02 bis 5, ganz besonders bevorzugt im Bereich von 0,05 bis 1.

[A7] In einer weiteren bevorzugten Ausführungsform der Frisur-stabilisierenden Zusammensetzung ist das Polyol B) ausgewählt aus der Gruppe bestehend aus Bernstein-, Methylbernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal-, Cyclohexandicarbon-, Malein-, Fumar-, Malon- oder Trimellitsäure sowie Säureanhydride, wie o-Phthal-, Trimellit- oder Bernsteinsäureanhydrid oder deren Gemische mit mehrwertigen Alkoholen, wie z.B. Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12 oder einer Mischung aus mindestens zwei hiervon.
Bei der Herstellung des bevorzugt amorphen Polyurethanharnstoffs wird eine aminofunktionelle Kettenverlängerer-Komponente C) mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist, eingesetzt.
Die aminofunktionellen Verbindungen der Komponente C) Komponente werden bevorzugt aus primären und/oder sekundären Diaminen ausgewählt. Insbesondere umfassen die aminofunktionellen Verbindungen C) mindestens ein Diamin.

[A8] In einer bevorzugten Ausführungsform der Frisur-stabilisierenden Zusammensetzung umfasst die aminofunktionelle Komponente C) wenigstens eine aminofunktionelle Verbindung C2), die ionische und/oder ionogene Gruppen aufweist. Die aminofunktionelle Verbindung C2) ist obligatorisch, wenn die Komponente D) nicht in der wässrigen Polyurethanharnstoff-Dispersion zur Herstellung des erfindungsgemäßen Frisur-stabilisierenden Zusammensetzung vorhanden ist.
Bevorzugt umfasst die aminofunktionelle Komponente C) sowohl aminofunktionelle Verbindungen C2), die ionische und/oder ionogene Gruppe aufweisen, als auch aminofunktionelle Verbindungen C1), die keine ionische oder ionogene Gruppe aufweisen.
Beispielsweise können als Komponente C1) organische Di- oder Polyamine wie beispielsweise 1,2-Ethylendiamin, 1,2- und 1,3-Diaminopropan, 1,4-Diaminobutan, 1,6-Diaminohexan, Isophorondiamin (IPDA), Isomerengemisch von 2,2,4- und 2,4,4-Trimethylhexamethylendiamin, 2-Methylpentamethylendiamin, Diethylentriamin, 4,4-Diaminodicyclohexylmethan und/oder Dimethylethylendiamin oder Mischungen aus mindestens zwei hiervon eingesetzt werden.
Bevorzugt ist die Komponente C1) ausgewählt aus der Gruppe bestehend aus 1,2-Ethylendiamin, Bis(4-aminocyclohexyl)methan, 1,4-Diaminobutan, IPDA, Ethanolamin, Diethanolamin und Diethylentriamin oder einer Mischung aus mindestens zwei hiervon.
Bevorzugt enthält die Komponente C1) > 75 mol%, besonders bevorzugt ≥ 80 mol%, ganz besonders bevorzugt ≥ 85 mol%, weiterhin bevorzugt ≥ 95 mol% und des Weiteren bevorzugt 100 mol% 1,2-Ethylendiamin oder IPDA oder ein Gemisch aus 1,2-Ethylendiamin und IPDA, wobei die Summe der beiden Amine in Bezug auf die Gesamtmenge an C1) bevorzugt in den für Komponente C1) genannten Anteilen vorliegt.
Bevorzugt umfasst die hydrophilierende Komponente C2) mindestens eine anionisch hydrophilierende Verbindung. Weiterhin bevorzugt beinhaltet die hydrophilierende Komponente C2) eine anionisch hydrophilierende Verbindung zu mindestens 80 Gew.-%, oder bevorzugt zu mindestens 90 Gew.-%, bezogen auf das Gesamtgewicht der Komponente C2). Besonders bevorzugt besteht die Komponente C2) aus ausschließlich anionisch hydrophilierenden Verbindungen.
Geeignete anionisch hydrophilierende Verbindungen enthalten wenigstens eine anionische oder ionogene Gruppe, die in eine anionische Gruppe überführt werden kann. Des Weiteren bevorzugt weisen geeignete anionisch hydrophilierende Verbindungen wenigstens zwei Aminogruppen und besonders bevorzugt zwei Aminogruppen auf. Besonders bevorzugt umfasst die hydrophilierende Komponente C2) eine anionisch hydrophilierende Verbindung, die wenigstens eine anionische oder ionogene Gruppe und wenigstens zwei Aminogruppen aufweist oder besteht daraus.
Geeignete anionisch hydrophilierende Verbindungen als Komponente C2), im Weiteren auch Hydrophilierungsmittel C2) genannt, enthalten bevorzugt eine Sulfonsäure- oder Sulfonatgruppe, besonders bevorzugt eine Natriumsulfonatgruppe. Geeignete anionisch hydrophilierende Verbindungen als Komponente C2) sind insbesondere die Alkalimetallsalze der Mono- und Diaminosulfonsäuren. Beispiele solcher anionischen Hydrophilierungsmittel sind Salze der 2-(2-Aminoethylamino)ethansulfonsäure, Ethylendiamin-propyl- oder -butylsulfonsäure oder 1,2- oder 1,3-Propylendiamin-β-ethylsulfonsäure oder Mischungen aus mindestens zwei hiervon.
Besonders bevorzugte anionische Hydrophilierungsmittel C2) sind solche, die Sulfonatgruppen als ionische Gruppen und zwei Aminogruppen enthalten, wie die Salze der 2-(2-Aminoethylamino)ethylsulfonsäure und 1,3-Propylendiamin-β-ethylsulfonsäure. Ganz besonders bevorzugt wird 2-(2-Aminoethylamino)ethylsulfonsäure oder deren Salze als anionisches Hydrophilierungsmittel C2) eingesetzt.
Gegebenenfalls kann die anionische Gruppe in der Komponente C2) auch eine Carboxylat- bzw. Carbonsäuregruppe

sein. Beispiele für Carbonsäuren sind 6-Aminohexansäure, Alanin, Asparaginsäure, Glutaminsäure, Glutamin, Glycin, Lysin, Leucin und Isoleucin oder Mischung aus mindestens zwei hiervon. Weitere Beispiele für geeignete Substanzen für die Komponente C2) sind bevorzugt aus Diaminocarbonsäuren ausgewählt. Als Beispiel für Diaminocarbonsäuren sei Lysin genannt.
Bei dieser alternativen Ausführungsform müssen jedoch die Carbonsäure-basierenden Komponenten C2) in höheren Konzentrationen eingesetzt werden, verglichen mit solchen Komponenten C2), die Sulfonat- oder Sulfonsäuregruppen tragen. Besonders bevorzugt werden daher zur Herstellung des bevorzugt amorphen Polyurethanharnstoffs keine hydrophilierenden Verbindungen, die ausschließlich Carboxylatgruppen als anionische Gruppen der Komponente C2) tragen eingesetzt.
Bevorzugt wird zur Herstellung des erfindungsgemäß verwendeten bevorzugt amorphen Polyurethanharnstoffs ≥ 0,1 und ≤ 10 Gew.-% der Komponente C2) und besonders bevorzugt ≥ 0,5 und ≤ 4 Gew.-% der Komponente C2), jeweils bezogen auf die Gesamtmasse des bevorzugt amorphen Polyurethanharnstoffs, eingesetzt.
Zur Hydrophilierung können auch Mischungen aus anionischen Hydrophilierungsmitteln C2) und weiteren Hydrophilierungsmitteln D), welche von C2) verschieden sind, verwendet werden.
Geeignete weitere Hydrophilierungsmittel D) sind beispielsweise nichtionische hydrophilierende Verbindungen D1) und/oder hydroxyfunktionelle ionische oder ionogene Hydrophilierungsmittel D2).

[A9] In einer bevorzugten Ausführungsform der Frisur-stabilisierenden Zusammensetzung handelt es sich bei der Komponente D) ausschließlich um nichtionisch hydrophilierende Komponenten D1).

Geeignete hydroxyfunktionelle ionische oder ionogene Hydrophilierungsmittel als Komponente D2) sind beispielsweise Hydroxycarbonsäuren wie Mono- und Dihydroxycarbonsäuren, wie 2-Hydroxyessigsäure, 3-Hydroxypropansäure, 12-Hydroxy-9-octadecansäure (Rizinolsäure), Hydroxypivalinsäure, Milchsäure, Dimethylolbuttersäure und/oder Dimethylolpropionsäure oder Gemische von mindestens zwei hieraus. Bevorzugt sind Hydroxypivalinsäure, Milchsäure und/oder Dimethylolpropionsäure, besonders bevorzugt ist Dimethylolpropionsäure. Bevorzugt werden keine hydroxyfunktionellen ionischen oder ionogenen Hydrophilierungsmittel D2), insbesondere bevorzugt keine Hydrophilierungsmittel, die Carboxylat und Hydroxygruppen aufweisen, wie beispielsweise Dimethylolpropionsäure eingesetzt. Bevorzugt ist die Menge an hydroxyfunktionellen ionischen oder ionogenen Hydrophilierungsmittel D2) in einem Bereich von 0 bis 1 Gew.-%, oder bevorzugt in einem Bereich von 0,01 bis 0,5 Gew.-%, bezogen auf die Gesamtmasse des bevorzugt amorphen Polyurethanharnstoffs in dem bevorzugt amorphen Polyurethanharnstoff enthalten.
Geeignete nichtionisch hydrophilierende Verbindungen als Komponente D1) sind z.B. Polyoxyalkylenether, welche über isocyanatreaktive Gruppen, wie Hydroxy-, Amino- oder Thiolgruppen verfügen. Bevorzugt sind monohydroxyfunktionelle, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38). Diese sind entweder reine Polyethylenoxidether oder gemischte Polyalkylenoxidether, wobei sie mindestens 30 mol-%, bevorzugt mindestens 40 mol-% bezogen auf alle enthaltenen Alkylenoxideinheiten an Ethylenoxideinheiten enthalten.
Besonders bevorzugte nichtionische Verbindungen sind monofunktionelle gemischte Polyalkylenoxidpolyether, die 40 bis 100 mol-% Ethylenoxid- und 0 bis 60 mol-% Propylenoxideinheiten aufweisen.
Geeignete Startermoleküle für solche nichtionischen Hydrophilierungsmittel sind insbesondere gesättigte Monoalkohole wie Methanol, Ethanol, n-Propanol, Isopropanol, n-Butanol, Isobutanol, sec-Butanol, die isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykol-monoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether, Methanol oder n-Butanol als Startermoleküle verwendet.
Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.
Bevorzugt enthält der bevorzugt amorphe Polyurethanharnstoff ≥ 0 und ≤ 20 Gew.-% der Komponente D), bevorzugt ≥ 0,1 und ≤ 10 Gew.-% der Komponente D) und ganz besonders bevorzugt ≥ 1 und ≤ 5 Gew.-% der Komponente D), jeweils bezogen auf die Gesamtmasse des bevorzugt amorphen Polyurethanharnstoffs. In einer weiteren bevorzugten Ausführungsform wird Komponente D) zur Herstellung des bevorzugt amorphen Polyurethanharnstoffs nicht verwendet.

In diesem Fall ist Komponente C2) zwingend in dem Polyurethanharnstoff enthalten.

Als Komponente E) können wahlweise Polyole, insbesondere nicht-polymere Polyole, des genannten Molekulargewichtsbereichs von 62 bis 399 mol/g mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A (2,2-Bis(4-hydroxycyclohexyl)propan), Trimethylolpropan, Trimethylolethan, Glycerin, Pentaerythrit sowie deren beliebige Mischungen untereinander eingesetzt werden.

Bevorzugt weist der bevorzugt amorphe Polyurethanharnstoff ≤ 10 Gew.-% der Komponente E), oder bevorzugt ≤ 5 Gew.-% der Komponente E), jeweils bezogen auf die Gesamtmasse des bevorzugt amorphen Polyurethanharnstoffs auf. Bevorzugt beinhaltet der bevorzugt amorphe Polyurethanharnstoff die Komponente E) in einem Bereich von 0,1 bis 10 Gew.-%, oder bevorzugt in einem Bereich von 0,2 bis 8 Gew.-%, oder bevorzugt in einem Bereich von 0,1 bis 5 Gew.-%, bezogen jeweils auf die Gesamtmasse des bevorzugt amorphen Polyurethanharnstoffs. In einer weiteren bevorzugten Ausführungsform wird Komponente E) zur Herstellung des bevorzugt amorphen Polyurethanharnstoffs nicht verwendet.

Bevorzugt wird zur Herstellung des bevorzugt amorphen Polyurethanharnstoffs ≥ 0,5 und ≤ 20 Gew.-% gebildet aus der Summe der Komponenten C1) und gegebenenfalls E) und besonders bevorzugt ≥ 1 und ≤ 15 Gew.-% gebildet aus der Summe der Komponenten C1) und gegebenenfalls E), jeweils bezogen auf die Gesamtmasse des bevorzugt amorphen Polyurethanharnstoffs, eingesetzt.

Als Komponente E) geeignete di- oder höherfunktionelle Polyolkomponenten mit einem Molekulargewicht von 62 bis 399 sind beispielsweise die unter B) aufgeführten Produkte, soweit sie ein Molekulargewicht von 62 bis 399 g/mol aufweisen. Weitere geeignete Komponenten sind die zur Herstellung der Polyesterpolyole genannten mehrwertigen, insbesondere zweiwertigen Alkohole sowie weiterhin niedermolekulare Polyesterdiole wie z.B. Adipinsäure-bis-(hydroxyethyl)-ester oder kurzkettige auf aromatischen Diolen gestartete Homo- und Mischadditionsprodukte des Ethylenoxid oder des Propylenoxid in Frage. Beispiele für aromatische Diole, die als Starter für kurzkettige Homo- und Mischpolymerisate des Ethylenoxid oder des Propylenoxid Verwendung finden können, sind z. B. 1,4-, 1,3-, 1,2-Dihydroxybenzol oder 2,2-Bis-(4-hydroxyphenyl)-propan (Bisphenol A).

Ferner können als Komponente F) auch monofunktionelle isocyanatreaktive Hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Diethylenglykolmonobutylether, Propylenglykolmonomethylether, Dipropylenglykolmonomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol, die auch als Mischungen von mindestens zwei der genannten eingesetzt werden können. Bevorzugt enthält die Frisur-stabilisierende Zusammensetzung weniger als 10 Gew.-% der Komponente F), oder bevorzugt weniger als 5 Gew.-% der Komponente F), oder bevorzugt in einem Bereich von 0,01 bis 5 Gew.-%, jeweils bezogen auf die Gesamtmasse der Frisur-stabilisierenden Zusammensetzung, noch bevorzugter wird Komponente F) zur Herstellung der Frisur-stabilisierenden Zusammensetzung nicht verwendet.

Die Frisur-stabilisierende Zusammensetzung enthält bevorzugt ein oder mehrere kosmetische Hilfssubstanzen V3), die in der Kosmetik üblich sind, wie Antioxidantien, Lichtschutzmittel und/oder andere Hilfs- und Zusatzmittel wie beispielsweise Emulgatoren, Benetzungsmittel wie grenzflächenaktive Stoffe, Weichmacher wie Glycerin, Glykol und Phthalester- und ether, Entschäumer, Verdicker und Rheologiemodifizierer, Geliermittel Antiklebmittel, Tenside, Wirkstoffe, Feuchthaltemittel, Füllstoff, UV-Filter, Filmbildner, Lösemittel, Koaleszenzmittel, Aromastoffe, Geruchsabsorber, Riechstoffe und Parfüms, Gelbildner und/oder andere Polymerdispersionen wie beispielsweise Dispersionen auf Basis von Polyacrylaten, Pigmente, Farbstoffe, antikorrosive Mittel, Neutralisationsmittel, Verlaufsmittel und/oder Thixotropiemittel, Geschmeidigkeitsmittel, Konservierungsmittel, Proteine und Derivate davon, Aminosäuren, Vitamine, Trübungsmittel, Stabilisatoren, Sequestierungsmittel, Komplexbildner, Perlglanzmittel, ästhetische Verstärker, Fettsäuren, Fettalkohole, Triglyceride, botanische Extrakte und Klärhilfsmittel. Die erfindungsgemäßen Zusammensetzungen weisen bevorzugt einen oder mehrere Emulgatoren bzw. oberflächenaktive Mittel auf.

Die Mengen der verschiedenen Zusatzstoffe sind dem Fachmann für den einzusetzenden Bereich der Haarkosmetischen, insbesondere der Frisur-stabilisierenden Zusammensetzungen, bekannt und liegen beispielweise im Bereich von 0 bis 25 Gew.-%, bevorzugt von 0 bis 15 Gew.-%, oder bevorzugt von 0,001 bis 15 Gew.-%, oder bevorzugt für jeden einzelnen Zusatzstoff jeweils in einem Bereich von 0,001 bis 5 Gew.-%, oder jeweils bevorzugt von 0,01 bis 3 Gew.-% bezogen auf das Gesamtgewicht der Zusammensetzung.

Bevorzugt beinhaltet die Frisur-stabilisierende Zusammensetzung weiterhin mindestens eine der folgenden Komponenten:

V3) mindestens eine kosmetisch aktive Substanzen und/oder ein oder mehrere kosmetische Hilfssubstanzen,

V4) mindestens ein Lösungsmittel oder Verdünner,

V5) mindestens einen zusätzlichen Filmbildner.

So enthalten bevorzugte Frisur-stabilisierende Zusammensetzung in Form von Öl-in-Wasser-Emulsionen (O/W) bevorzugt mindestens einen Emulgator mit einem HLB-Wert > 7 und gegebenenfalls einen Coemulgator.

Vorteilhaft werden die folgenden nichtionischen Emulgatoren eingesetzt:

a) Partialfettsäureester und Fettsäureester mehrwertiger Alkohole und deren ethoxylierte Derivate (z. B. Glycerylmonostearat, Sorbitanstearat, Glyceryl-Stearyl-Citrat, Sucrose-Stearat)

b) ethoxylierte Fettalkohole und Fettsäuren.

Besonders vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder Triethanolamin-Salze der Stearin- oder Palmitinsäure) sowie Ester der Zitronensäure wie Glycerylstearatcitrat.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemässen Zubereitungen weiter erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusoel, Polyglyceryl-4-isostearat, Acrylat/$C_{10-30}$-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

Die Frisur-stabilisierenden Zusammensetzungen enthalten bevorzugt Verdicker, insbesondere in der Wasserphase beispielsweise in einer O/W-Zusammensetzung. Vorteilhafte Verdicker sind:

- Vernetzte oder nichtvernetzte Acrylsäure- oder Methacrylsäure-Homo- oder Copolymere. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate und Vinylacetat.

- Verdickende Polymere natürlicher Herkunft beispielweise auf Cellulosebasis, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan.

- Nichtionische, anionische, kationische oder amphotere assoziative Polymere z.B. auf Basis von Polyethylenglycole und ihre Derivate, oder Polyurethane.

- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, vernetzte Acrylsäure oder Methacrylsäure Homo- oder Copolymere und vernetzte Copolymere von 2-Acrylamido-2-methylpropansulfonsäure.

Ganz besondere vorteilhafte Verdicker sind Xanthangummi, wie die unter den Bezeichnungen Keltrol® und

Kelza® von der Firma CP Kelco angebotenen Produkte oder die Produkte der Firma RHODIA mit der Bezeichnung Rhodopol® und Guargummi, wie die unter der Bezeichnung Jaguar® HP105 von der Firma RHODIA erhältlichen Produkte.

Ganz besondere vorteilhafte Verdicker sind auch vernetzte Homopolymere von Methacrylsäure oder Acrylsäure, die von der Firma Lubrizol unter den Bezeichnungen Carbopol® 940, Carbopol® 941, Carbopol® 980, Carbopol® 981, Carbopol® ETD 2001, Carbopol® EDT 2050, Carbopol® 2984, Carbopol® 5984 und Carbopol® Ultrez 10, von der Firma 3V unter den Bezeichnungen Synthalen® K, Synthalen® L und Synthalen® MS und von der Firma PROTEX unter den Bezeichnungen Modarez® V 1250 PX, Modarez® V2000 PX, Viscaron® A1600 PE und Viscaron® A700 PE im Handel erhältlich sind.

Ganz besondere vorteilhafte Verdicker sind vernetzte Copolymer von Acrylsäure oder Methacrylsäure und einem $C_{10-30}$-Alkylacrylat oder $C_{10-30}$-Alkylmethacrylat und Copolymere von Acrylsäure oder Methacrylsäure und Vinylpyrrolidon. Solche Copolymere sind beispielsweise von der Firma Lubrizol unter den Bezeichnungen Carbopol® 1342, Carbopol® 1382, Pemulen® TR1 oder Pemulen® TR2 und von der Firma ISP unter den Bezeichnungen Ultrathix® P-100 (INCI : Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex® AVC (INCI: Ammonium Acryloyldimethyltaurate/VP Copolymer) erhältlich.

Falls die Verdicker eingesetzt werden, sind die Verdicker im Allgemeinen in einer Konzentration von etwa 0 % bis 2 Gew.-% vorzugsweise 0 % bis 1 Gew.-% oder bevorzugt in einem Bereich von 0,01 bis 1 Gew.-% bezogen auf das Gesamtgewicht der erfindungsgemäßen Zusammensetzung vorhanden.

Die bevorzugten Lösungsmittel als Komponente V4) sind beispielsweise die aliphatischen Alkohole mit C1-4 Kohlenstoffatomen wie Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, und deren Gemische.

Der Mengenanteil des Lösungsmittels oder der Lösungsmittel V4) in der erfindungsgemäßen Zusammensetzung kann beispielsweise im Bereich von 0 bis 25 Gew.-%, oder bevorzugt 0 bis 15 Gew.-%, bezogen auf das Gesamtgewicht der Zusammensetzung, liegen.

Die erfindungsgemäßen Zusammensetzungen können des weiteren ein Treibgas enthalten.

Bevorzugte Treibgase sind Kohlenwasserstoffe wie Propan, Isobutan und n-Butan sowie deren Mischungen. Druckluft, Kohlendioxid, Stickstoff, Stickstoffdioxid und Dimethylether sowie Mischungen all dieser Gase können ebenfalls verwendet werden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte. Dabei handelt es sich, insbesondere Fluorkohlenwasserstoffe und Fluorchlorkohlenwasserstoffe (FCKW) wie z.B. 1,2-difluoroethan (Treibmittel 152 A).

Bevorzugt sind in der Frisur-stabilisierenden Zusammensetzung des weiteren haarpflegende Wirkstoffe enthalten. Als Pflegestoffe können bevorzugt cyclische Polydimethylsiloxane (Cyclomethicone) oder Silikontenside (Polyethermodifizierte Siloxane) vom Typ Dimethicone Copolyol oder Simethicon zum Einsatz kommen. Cyclomethicone werden u. a. unter der Handelsbezeichnungen Abil® K4 von der Firma Goldschmidt oder z.B. DC 244, DC 245 oder DC 345 von der Firma Dow Corning angeboten. Dimethicon-Copolyole werden z. B. unter der Handelsbezeichnung DC 193 von der Firma Dow Corning bzw. Belsil® DM 6031 von der Firma Wacker angeboten.

*Tenside*

Die erfindungsgemäßen Zusammensetzungen können auch Tenside enthalten, die aus der Gruppe von anionischen, kationischen, nichtionischen und/oder amphoteren Tensiden ausgewählt werden.

Vorteilhafte anionische Tenside im Sinne der vorliegenden Erfindung sind:

- Acylaminosäuren und deren Salze, wie Acylglutamate, insbesondere Natriumacylglutamat und Sarcosinate, beispielsweise Myristoyl Sarcosin, TEA-Lauroyl Sarcosinat, Natriumlauroylsarcosinat und Natriumcocoylsarkosinat,
- Sulfonsäuren und deren Salze, wie Acylisethionat, beispielsweise Natrium- oder Ammoniumcocoylise-thionat, Sulfosuccinate, beispielsweise Dioctylnatriumsulfosuccinat, Dinatriumlaurethsulfosuccinat, Dinatriumlaurylsulfosuccinat und Dinatriumundecylenamido MEA- Sulfosuccinat, Dinatrium PEG-5 Laurylcitratsulfosuccinat und Derivate;
- Schwefelsäureester, wie Alkylethersulfat, beispielsweise Natrium-, Ammonium-, Magnesium-, MIPA-, TIPALaurethsulfat, Natriummyrethsulfat und Natrium $C_{12}$ bis $C_{13}$ - Parethsulfat, und Alkylsulfate, beispielsweise Natrium-, Ammoniumund TEA-Laurylsulfat,
- Taurate, beispielsweise Natriumlauroyltaurat und Natriummethylcocoyltaurat;

- Ether-Carbonsäuren, beispielsweise Natriumlaureth-13 Carboxylat und Natrium PEG-6 Cocamide Carboxylat, Natrium PEG-7-Olivenöl-Carboxylat;
- Phosphorsäureester und Salze, wie beispielsweise DEA-Oleth-10 Phosphat und Dilaureth-4 Phosphat;
- Alkylsulfonate, beispielsweise Natriumcocosmonoglyceridsulfat, Natrium $C_{12}$ bis $C_{14}$ - Olefinsulfonat, Natriumlaurylsulfoacetat und Magnesium PEG-3 Cocamidsulfat,
- Acylglutamate wie Di-TEA-palmitoylaspartat und Natrium Caprylic/Capric Glutamat;
- Acylpeptide, beispielsweise Palmitoyl hydrolysiertes Milchprotein, Natrium Cocoyl hydrolysiertes Sojaprotein und NatriunWKalium Cocoyl hydrolysiertes Kollagen;
- Carbonsäuren und Derivate, beispielsweise Laurinsäure, Aluminiumstrearat, Magnesiumalkanolat und Zinkundecylenat, Ester-Carbonsäuren, beispielsweise Calciumstearoyilactylat, Laureth-6 Citrat und Natrium PEG-4 Lauramidcarboxylat;
- Alkylarylsulfonate.

Vorteilhafte kationische Tenside im Sinne der vorliegenden Erfindung sind quaternäre Tenside. Quaternäre Tenside enthalten mindestens ein N-Atom, das mit 4 Alkyl-, oder Arylgruppen kovalent verbunden ist. Vorteilhaft sind beispielsweise Alkylbetain, Alkylamidopropylbetain und Alkylamidopropylhydroxysultain.

Weitere vorteilhafte kationische Tenside im Sinne der vorliegenden Erfindung sind ferner Alkylamine, Alkylimidazole und ethoxylierte Amine und insbesondere deren Salze.

Vorteilhafte amphotere Tenside im Sinne der vorliegenden Erfindung sind Acyl-/ dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-Kokosfettsäureamidoethyl-N-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte aktive nicht-ionische Tenside im Sinne der vorliegenden Erfindung sind Alkanolamide, wie Cocamide MEA/DEA/MIPA, Ester, die durch Veresterung von Carbonsäuren mit Ethylenoxid, Glycerin, Sorbitan oder anderen Alkoholen entstehen, Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Belsil®SPG 128V der Firma Wacker).

Weitere vorteilhafte nicht-ionische Tenside sind Alkohole und Aminoxide, wie Cocoamidopropylamin-Oxid.

Unter den Alkylethersulfaten sind insbesondere Natriumalkylethersulfate auf Basis von zwei oder dreifach ethoxyliertem Laurylund Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Haut und Schleimhautverträglichkeit. Laurylethersulfat weist bessere Schaumeigenschaften als Myristylethersulfat auf, ist diesem aber in der Milde unterlegen.

Alkylethercarboxylate mit mittlerem und besonders mit höherem gehören zu den mildesten Tensiden überhaupt, zeigen aber ein schlechtes Schaum und Viskositätsverhalten. Sie werden oft in Kombination mit Alkylethersulfaten und amphoteren Tensiden eingesetzt.

Sulfobemsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt.

Amidopropylbetaine weisen eine ausgezeichnete Haut und Augenschleimhautverträglichkeit auf. In Kombination mit anionischen Tensiden lässt sich deren Milde synergistisch verbessern. Bevorzugt ist die Verwendung von Cocamidopropylbetain.

Arnphoacetate/Amphodiacetate besitzen als amphotere Tenside eine sehr gute Haut und Schleimhautverträglichkeit und können konditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind mild, haben gute Universaleigenschaften, schäumen jedoch schwach. Aus diesem Grund werden sie bevorzugt in Kombinationen mit anionischen Tensiden verwendet.

*Konditionierungsmittel*

Gegebenenfalls enthalten die erfindungsgemäßen Zusammensetzungen ein Konditionierungsmittel. Bevorzugte Konditionierungsmittel sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, Skin-Conditioning Agents, SkinConditioning Agents-Emollient, Skin-Conditioning Agents-Humectant, SkinConditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble conditioning agent" aufgeführten Verbindungen.

Besondere vorteilhafte Konditionierungsstoffe stellen beispielsweise die nach INCI als Polyquaternium bezeichneten Verbindungen dar (insbesondere Polyquaternium-1 bis Polyquaternium-114).

Zu den geeigneten Konditionierungsmitteln zählen beispielsweise auch polymere quaternäre Ammoniumverbindungen, kationische Cellulosederivate, Chitosanderivate Guar Gum Derivate und Polysaccharide, insbesondere Guar Hydroxypropylammoniumchlorid (z.B. Jaguar® Excel, Jaguar®162 der Firma Rhodia).

Weitere erfindungsgemäß vorteilhafte Konditionierungsmittel stellen nichtionische Poly-N-vinylpyrrolidon/Polyvinylacetat-Copolymere (z.B. Luviskol®VA 64 der Firma BASF AG), anionische Acrylat-Copolymere (z.B. Luviflex®Soft der Firma BASF AG), und/oder amphotere Amid/Acrylat/Methacrylat Copolymere (z.B. Amphomer® der Firma National Starch dar. Weitere mögliche Konditioniermittel sind quaternisierte Silikone.

Optional können herkömmliche Additive ebenfalls in der Zusammensetzung enthalten sein, beispielsweise um ihr bestimmte Modifizierungseigenschaften zu verleihen. Hierbei kann es sich etwa um Silikone oder Silikonderivate, Benetzungsmittel, Feuchthaltemittel, Weichmacher wie Glycerin, Glykol und Phthalester und -ether, Riechstoffe und Parfüms, UV-Absorber, Farbstoffe, Pigmente, und andere Farbmittel, antikorrosive Mittel, Neutralisationsmittel, Antioxidantien, Antiklebemittel, Kombinierungsmittel und Konditioniermittel, antistatische Mittel, Glanzmittel, Konservierungsmittel, Proteine und Derivate davon, Aminosäuren, Vitamine, Emulgatoren, oberflächenaktive Mittel, Viskositätsmodifizierer, Verdicker und Rheologiemodifizierer, Geliermittel, Trübungsmittel, Stabilisatoren, Tenside, Sequestierungsmittel, Komplexbildner, Perlglanzmittel, ästhetische Verstärker, Fettsäuren, Fettalkohole, Triglyceride, botanische Extrakte, Klärhilfsmittel und Filmbildner handeln.

Diese Additive sind im Allgemeinen in einer Konzentration von etwa 0,001 % bis 15 Gew.-% vorzugsweise 0,01 % bis 10 Gew.-% bezogen auf das Gesamtgewicht der Zusammensetzung vorhanden.

Bevorzugt liegt in der Frisur-stabilisierenden Zusammensetzung mindestens ein zusätzlicher Filmbildner der Komponente V5) ausgewählt aus der Gruppe bestehend aus einem nicht-ionischen, einem anionischen, einem amphoteren und/oder einem kationischen Polymer, vor.

Bevorzugt ist der Filmbildner V5) ausgewählt aus der Gruppe bestehend aus einem Polyacrylat, einem Polyacrylamid, einem Polyurethan, einem Polyharnstoff, einem Polysiloxan oder einer Mischung aus mindestens zwei hiervon. Zu den Polyacrylaten werden auch Silikon-Acrylat-Copolymere gezählt.

Unter Anderem zur Verbesserung der Beständigkeit von dekorativen Produkten gegenüber Wasser, Tränen oder Schweiß (oft Wasserfestigkeit genannt) werden filmbildende Polymere als Filmbildner eingesetzt.

Als filmbildende Polymere werden bevorzugt Polymere auf Basis von Acrylaten oder Vinylpyrrolidonen gewählt. Vorteilhafte Filmbildner sind Trimethylsiloxysilicate, Silikon Acrylate Copolymere (z. B. TIB4-200 der Fa. Dow Corning oder KP-561 der Fa. Shin Etsu), Trimethyl Pentaphenyl Trisiloxane (Dow Corning 555 Cosmetic Fluid der Fa. Dow Corning Ltd.) oder Vinylpyrrolidon-Copolymer (z.B. PVP/Eicosen-copolymer oder PVP/hexadecan-copolymer).

Bevorzugt kommen generell nicht-ionische, anionische, amphotere und/oder kationische Polymere als Filmbildner zum Einsatz. Bevorzugt nicht-ionische Polymere, die in der Frisur-stabilisierenden Zusammensetzung entweder alleine oder in Kombination bevorzugt mit anionischen und/oder amphoteren und/oder zwitterionischen Polymeren verwendet werden sind bevorzugt ausgewählt aus der Gruppe bestehend aus:

- Polyalkyloxazoline;
- Vinylacetat Homo- oder Copolymerisate, wobei. hierzu beispielweise Copolymerisate aus Vinylacetat und Acrylsäureester, Copolymerisate aus Vinylacetat und Ethylen, Copolymerisate aus Vinylacetat und Maleinsäureester gehören;
- Acrylsäureester Copolymerisate wie z. B. die Copolymerisate aus Alkyl-Acrylat und AlkylMethacrylat, Copolymerisate aus Alkyl-Acrylat und Urethanen;
- Copolymerisate aus Acrylnitril und nichtionischem monomer ausgewählt aus Butadien und (Meth)Acrylat;
- Styrol Homo- und Copolymerisate, wobei hierzu beispielweise Homopolystyrol, Copolymerisate aus Styrol und Alkyl-(Meth)Acrylat , Coplymerisate aus Styrol, AlkylMethacrylat und Alkyl-Acrylat, Copolymerisate aus Styrol und Butadien, Copolymerisate aus Styrol, Butadien und Vinylpyridin gehören;
- Polyamide;
- Vinyllactame Homo- oder Copolymere, wie Vinylpyrrolidon-Homo- oder Copolymerisate; wobei hierzu beispielweise

Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat gehören;

- Polysiloxane;
- Homopolymere des N-Vinylformamids wie PVF von AkzoNobel;
- Polyimide, wie Polyimide-1 in Styleze™ XT3 von Ashland, und
- Polyurethane, wie Baycusan® C2000 von Covestro Deutschland AG; oder
- Mischungen von mindestens zwei der Vorgenannten.

Besondere bevorzugte nichtionische Polymere sind Acrylsäureester Copolymerisate, Homopolymere des Vinylpyrrolidons und Copolymere sowie Polyvinylcaprolactam.

Besonders bevorzugte nichtionische Polymere sind Homopolymere des Vinylpyrrolidons z. B. Luviskol® K der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte anionische Polymere sind Homo-oder Copolymere mit Säuregruppen enthaltenden Monomereinheiten, die gegebenenfalls mit Comonomeren, die keine Säuregruppen enthalten copolymerisiert sind. Geeignete Monomere sind ungesättigte, radikalisch polymerisierbare Verbindungen, die mindestens eine Säuregruppe besitzen, und insbesondere Carbonsäure, Sulfonsäure oder Phosphonsäure.

Beispiele für anionische Polymere, die Carbonsäuregruppen enthalten sind:

- Acrylsäure oder Methacrylsäure Homo- oder Copolymere oder deren Salze. Hierzu gehören beispielweise die Copolymerisate aus Acrylsäure und Acrylamide und / oder deren Natriumsalze, Copolymerisate aus Acrylsäure und / oder Methacrylsäure und einem ungesättigten Monomer ausgewählt aus der Gruppe Ethylene, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone® LM der Firma ISP, Copolymerisate aus Methacrylsäure, Ethylacrylate und Tert-butyl Acrylate z.B. Luvimer® 100 P der Firma BASF;
- Crotonsäurederivat-Homo- oder Copolymere oder deren Salze. Hierzu gehören beispielweise Vinylacetat / Crotonsäure-, Vinylacetat/Acrylat- und / oder Vinylacetat / Vinylneodecanoat / Crotonsäure-Copolymere z.B. Resyn® 28-1310 oder Resyn® 28-2930 der Firma AkzoNobel oder Luviset® CAN der Firma BASF, Natriumacrylat/Vinylalkohol-Copolymere;
- ungesättigte C4-C8 Carbonsäurederivate oder Carbonsäureanhydrid Copolymere ausgewählt aus Copolymerisaten aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus der Gruppe Allylester, Methallylester und ggfs. Acrylamide, Methacrylamide, alpha-Olefin, Acrylsäureester, Methacrylsäureester, Vinylpyrrolidone. Weitere bevorzugte Polymere sind Methylvinylether/Maleinsäure-Copolymere, die durch Hydrolyse von Vinylether / Maleinsäureanhydrid-Copolymeren entstehen. Diese Polymere können auch teilverestert (Ethyl, Isopropyl- bzw. Butylester) oder teilamidiert sein;
- in Wasser lösliche oder dispergierbare anionische Polyurethane, z. B. Luviset® P.U.R. der BASF, und Dynamx® von AkzoNobel, Baycusan® C1000, Baycusan® C1001, Baycusan® C1003, Baycusan® C1004, Baycusan® C1008 von Covestro Deutschland AG,

Diese vorangehende Liste ist nicht abschließend und damit nicht einschränkend zu sehen.

Vorteilhafte anionische Polymere enthaltend Sulfonsäuregruppe sind Salze von Polyvinylsulfonsäuren, Polystyrolsulfonsäuren wie z. B. Natriumpolystyrolsulfonat oder von Polyacrylamidesulfonsäuren.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure

bzw. Itaconsäureanhydrid und mindestens einem Monomer ausgewählt aus der Gruppe Vinylester, Vinylether, Vinylhalogenderivate, Phenylvinylderivate, Acrylsäure, Acrylsäureester und Salze von Polystyrolsulfonsäuren.

Ganz besonders vorteilhafte anionische Polymere sind Acrylat-Copolymere, z.B. Luvimer® der BASF, Ethylacrylat / N-tert.-Butylacrylamid / Acrylsäure-Copolymere ULTRAHOLD® STRONG der BASF, VA / Crotonat / Vinylneodecanoat-Copolymer z. B. Resyn® 28-2930 der AkzoNobel, Copolymerisate wie. Z. Copolymerisate aus Methylvinylether und Maleinsäureanhydrid teilverestert z.B. GANTREZ® von Ashland und Natrium-polystyrolsulfonate, wie Flexan® 130 von AkzoNobel.

Vorteilhafte amphotere Polymere können unter den Polymeren ausgewählt werden, die statistisch in der Polymerkette verteilte Einheiten A und B enthalten, wobei A für eine Einheit steht, die von einem Monomer mit mindestens einem basischen Stickstoffatom abgeleitet ist, und B eine Einheit darstellt, die von einem sauren Monomer stammt, das eine oder mehrere Carboxy- oder Sulfonsäuregruppen aufweist. Alternativ können A und B Gruppen sein, die von zwitterionischen Carboxybetainmonomeren oder Sulfobetainmonomeren abgeleitet sind. A und B können auch eine kationische Polymerkette sein, die primäre, sekundäre, tertiäre oder quartanäre Gruppen enthalten, wobei mindestens eine Aminogruppe eine Carboxygruppe oder Sulfonsäuregruppe trägt, die über eine Kohlenwasserstoffgruppe gebunden ist, oder A und Bsind Teil einer Polymerkette mit Ethylen-$\alpha,\beta$-dicarbonsäureeinheit, bei der die Carbonsäuregruppen mit einem Polyamin umgesetzt wurden, das eine oder mehrere primäre oder sekundäre Aminogruppen enthält.

Bevorzugte amphotere Polymere sind:

- Polymere, die bei der Copolymerisation eines von einer Vinylverbindung mit Carboxygruppe abgeleiteten Monomers, wie insbesondere Acrylsäure, Methacrylsäure, Maleinsäure, $\alpha$-Chloracrylsäure, und einem basischen Monomer gebildet werden. Das basische Monomer ist von einer Vinylverbindung abgeleitet, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent US 3,836,537 beschrieben worden.

- Polymere mit Einheiten, die von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat abgeleitet sind.
  Besonders bevorzugte N-substituierte Acrylamide oder Methacrylamide sind Verbindun-gen, deren Alkylgruppen 2 bis 12 Kohlenstoffatome enthalten. Ganz besonders bevorzugt sind N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.
  Geeignete saure Comonomere sind insbesondere aus der Gruppe Acrylsäure, Methacryl-säure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.
  Bevorzugte basische Comonomere sind Aminoethylmethacrylat, Butylaminoethylmethacrylat, N,N-Dimethylaminoethylmethacrylat, N-t-Butylaminoethylmethacrylat..

- Vernetzte und ganz oder teilweise acylierte Polyaminoamide, die von Polyaminoamiden der folgenden allgemeinen Formel:

  -[CO-R-CO-Z]-

  abgeleitet sind, worin R eine zweiwertige Gruppe ist, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bis-sekundäres Amin entsteht, und Z eine Gruppe ist, die von einem bis-primären, mono- oder bis-sekundären Polyalkylenpolyamin abgeleitet ist, und bevorzugt: a) als quantitative Fraktion von 60 bis 100 mol% die Gruppen $-NH-[(CH_2)_x-NH-]p-$ wobei x = 2 und p = 2 oder 3 oder x = 3 und p = 2, wobei die Gruppe gebildet wird von Diethylentriamin, Triethylentetramin oder Dipropylentriamin; b) als quantitative Fraktion von 0 bis 40 mol% die Gruppe $-NH-[(CH_2)_x-NH-]p-$, wobei x = 2 und p = 1, das erhältlich ist aus Ethylenediamin, oder der Gruppe, die von Piperazin stammt:

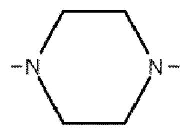

c) in quantitativer Fraktion von 0 bis 20 mol.-%, die Gruppe $-H-(CH_2)_6-NH-$, das erhältlich ist aus Hexamethylenediamin, wobei diese Polyaminoamide vernetzt sind durch Addition eines bifunktionalen Vernetzers, bevorzugt ausgewählt aus der Gruppe von Epihalohydrinen, Diepoxiden, Dianhydriden und bis-ungesättigten Derivaten, in einer Menge in einem Bereich von 0.025 bis 0.35 mol von Vernetzer pro Aminogruppe des Polyaminoamids, und acyliert mit Acrylsäure, Chloressigsäure oder einem Alkansulfon oder Salzen davon.
Die gesättigten Carbonsäuren sind vorzugsweise unter den Säuren mit 6 bis 10 Kohlenstoffatomen, wie Adipinsäure, 2,2,4-Trimethyladipinsäure und 2,4,4,-Trimethyladipinsäure, Terephthalsäure; Säuren mit ethylenischer Doppelbindung, wie beispielsweise Acrylsäure,
Die bei der Acylierung verwendeten Alkansultone sind vorzugsweise Propansulton oder Butansulton, die Salze der Acylierungsmittel sind vorzugsweise die Natriumsalze oder Kaliumsalze.

- Polymere mit zwitterionischen Einheiten der folgenden Formel:

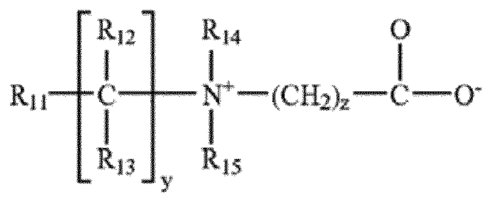

worin $R_{11}$ eine polymerisierbare ungesättigte Gruppe, wie Acrylat, Methacrylat, Acrylamid oder Methacrylamid, y und z ganze Zahlen von 1 bis 3, $R_{12}$ und $R_{13}$ ein Wasserstoffatom, Methyl, Ethyl oder Propyl, $R_{14}$ und $R_{15}$ ein Wasserstoffatom oder eine Alkylgruppe bedeuten, die so gewählt ist, dass die Summe der Kohlenstoffatome $R_{14}$ und $R_{15}$ 10 nicht übersteigt, sind.
Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.

- Polymere, die von Chitosan abgeleitet sind und Monomereinheiten enthalten, die den folgenden Formeln entsprechen:

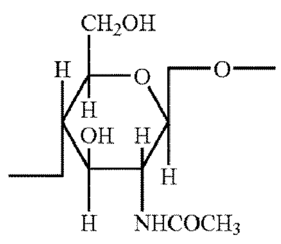

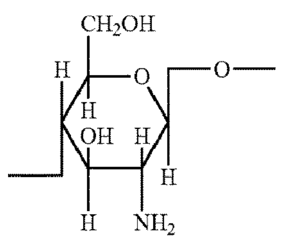

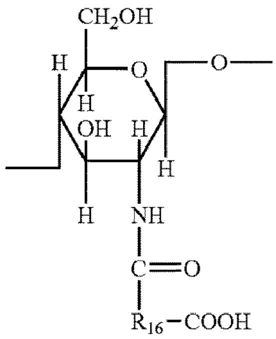

wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit $R_{16}$ eine Gruppe der folgenden Formel bedeutet:

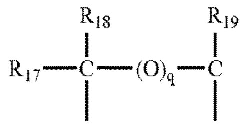

worin falls q = 0, die Gruppen $R_{17}$, $R_{18}$ und $R_{19}$, gleich oder verschieden jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und / oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen $R_{17}$, $R_{18}$ und $R_{19}$ in diesem Fall ein Wasserstoffatom bedeutet; oder falls q = 1, die Gruppen $R_{17}$, $R_{18}$ und $R_{19}$ jeweils ein Wasserstoffatom, sowie die Salze, die diese Verbindungen mit Basen oder Säuren bilden, sind.

- Polymere, die der folgenden allgemeinen Formel entsprechen und die beispielsweise in dem französischen Patent 1 400 366 beschrieben sind:

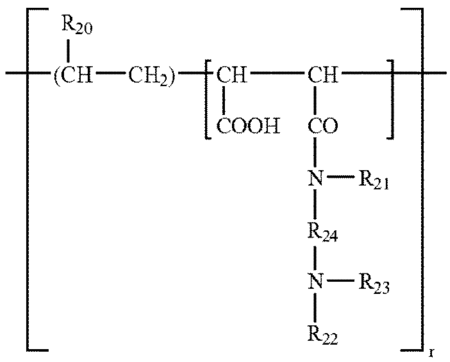

worin $R_{20}$ ein Wasserstoffatom, $CH_3O$, $CH_3CH_2O$ oder Phenyl ist, $R_{21}$ ein Wasserstoffatom oder eine niedere Alkylgruppe, wie Methyl oder Ethyl ist, $R_{22}$ ein Wasserstoffatom oder eine niedere $C_{1-6}$-Alkylgruppe, wie Methyl oder Ethyl ist, $R_{23}$ eine niedere $C_{1-6}$-Alkylgruppe, wie Methyl oder Ethyl oder eine Gruppe der Formel: $-R_{24}-N(R_{22})_2$ ist, wobei $R_{24}$ eine Gruppe $-CH_2-CH_2$, - $CH_2-CH_2-CH_2-$ oder $-CH_2-CH(CH_3)-$ darstellt und wobei $R_{22}$ die oben angegebenen Bedeutungen aufweist.

- Polymere, die bei der N-Carboxyalkylierung von Chitosan gebildet werden können, wie N-Carboxymethylchitosan oder N-Carboxybutylchitosan.

Alkyl($C_{1-5}$)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besonders vorteilhafte amphotere Polymere sind z.B. die Copolymere Octylacrylamid / Acrylate / Butylamino-Ethylmethacrylat-Copolymer, die unter den Bezeichnungen AMPHOMER®, AMPHOMER® LV 71 oder BALANCE® 47 der Firma AkzoNobel im Handel sind, und Methylmethacrylat / Methyl-dimethylcarboxymethylammoniumethylmethacrylat-Copolymere.

Es ist gegebenenfalls vorteilhaft, die anionischen und amphoteren Polymere zur Verbesserung ihrer Wasserlöslichkeit bzw. ihrer Wasserdispergierbarkeit mit geeigneten Basen zu neutralisieren.

Als Neutralisationsmittel für Polymere, die Säuregruppen enthalten, können folgende Basen eingesetzt werden: Hydroxide, deren Kation Ammonium oder ein Alkalimetall ist wie z. B. NaOH oder KOH.

Andere Neutralisationsmittel sind primäre, sekundäre oder tertiäre Amine, Aminoalkohole oder Ammoniak. Bevorzugt werden hier 2-Amino-2-methyl-1,3-propandiol (AMPD), 2-Amino-2-ethyl-1,3-propandiol (AEPD), 2-Amino-2-methyl-1-propanol (AMP), 2-Amino-1-butanol (AB), 2-Amino-1,3-propandiol, Monoethanolamin (MEA), Diethanolamin (DEA), Triethanolamin (TEA), Monoisopropanolamin (MIPA), Diisopropanolamin (DIPA), Triisopropanolamin (TIPA), Dimethyl Laurylamin (DML), Dimethyl Myristalamin (DMM), und Dimethyl Stearamin (DMS) verwendet.

Die Neutralisation kann je nach Anwendungszweck teilweise oder vollständig erfolgen.

Gegebenenfalls einsetzbar aber jedoch weniger bevorzugt sind kationische Polymere, wie beispielweise Polymere, die primäre, sekundäre tertiäre und / oder quaternäre Aminogruppen enthalten, die Teil der Polymerkette oder direkt an die Polymerkette gebunden sind.

Bevorzugt ist der zusätzliche Filmbildner V5) ein Polyurethan, das erhältlich ist durch Umsetzung eines oder mehrerer wasserunlöslicher, nicht wasserdispergierbarer, isocyanatfunktioneller Polyurethanprepolymere mit einer oder mehreren aminofunktionellen Verbindungen B), wobei B) aus den gleichen Verbindungen ausgewählt werden kann wie für B) oben beschrieben.

Im Rahmen der Erfindung bedeutet der Begriff "wasserunlösliches, nicht wasserdispergierbares Polyurethanprepolymer" insbesondere, dass die Wasserlöslichkeit des erfindungsgemäß verwendeten Prepolymers bei 23 °C weniger als 10 g / Liter, bevorzugter weniger als 5 g / Liter beträgt und das Prepolymer bei 23° keine sedimentationsstabile Dispersion in Wasser, insbesondere entionisiertem Wasser, ergibt. Mit anderen Worten setzt sich das Prepolymer, beim Versuch es in Wasser zu dispergieren, ab.

Bevorzugt ist das NCO-terminierte Polyurethanprepolymer erhältlich aus der Reaktion einer Reaktionsmischung umfassend ein Polyisocyanat und Polyol. Das Polyisocyanat weist bevorzugt eine Funktionalität in einem Bereich von >1,5 bis 6, oder bevorzugt von 1,8 bis 5, oder bevorzugt von 2 bis 4, insbesondere von 2 auf. Geeignete Polyisocyanate sind aliphatische, aromatische araliphatische oder cycloaliphatische Polyisocyanate. Beispiele solcher geeigneten Polyisocyanate sind 1,4-Butylendiisocyanat, 1,6-Hexamethy-lendiisocyanat (HDI), Isophorondiisocyanat (IPDI), 2,2,4- und/oder 2,4,4-Trimethyl¬hexa¬methy-len¬¬diiso¬cyanat, die isomeren Bis-(4,4'-isocyanatocyclo¬hexyl)¬methane oder deren Mischungen beliebigen Isomeren¬gehalts, 1,4-Cyclohexylendi¬isocyanat, 4-Isocyanatomethyl-1,8-octandiisocyanat (Nonantriisocyanat), 1,4-Phenylen¬di¬iso¬cya¬nat, 2,4- und/oder 2,6-Toluylendiisocyanat, 1,5-Naphthylenidiisocyanat, 2,2'-und/oder 2,4'- und/oder 4,4'-Di-phenylmethandiisocyanat, 1,3- und/oder 1,4-Bis-(2-isocyanato-prop-2-yl)-benzol (TMXDI), 1,3-Bis(isocyanato-methyl)benzol (XDI) sowie Alkyl-2,6-diisocyanatohexanoate (Lysin-diisocyanate) mit C1 bis C8-Alkylgruppen.

Bevorzugt ist das Polyisocyanat ein aliphatisches Polyisocyanat. Bevorzugte aliphatische Diisocyanate sind Hexamethylendiisocyanat und Isophorondiisocyanat sowie deren Gemische.

Das Polyol weist bevorzugt eine Funktionalität von >1,5 bis 6 oder bevorzugt von 1,8 bis 5, oder bevorzugt von 2 bis 4, insbesondere von 2 auf. Bevorzugt ist das Polyol ausgewählt aus der Gruppe bestehend aus einem Polyetherpolyol, einem Polycarbonatpolyol, einem Polyetherpolycarbonatpolyol, einem Polyesterpolyol oder einem Gemisch aus mindestens zwei hiervon. Bevorzugt weist das Polyol ein Polyoxyethylengruppen enthaltendes Polyol auf. Hinsichtlich der Polyole sind Copolymere aus Ethylenoxid und Propylenoxid mit einem Ethylenoxidgehalt, bezogen auf die Gesamtmenge der enthaltenen Oxyalkylengruppen, von 60 bis 85 mol-% bevorzugt. Weiterhin bevorzugt sind Polyesterpolyole bevorzugt gebildet aus einer Säure ausgewählt aus der Gruppe bestehend aus Malonsäure, Bernsteinsäure, Glutarsäure, Adipinsäure, Pimelinsäure, Korksäure, Azelainsäure, Sebacinsäure oder einer Mischung aus mindestens zwei hiervon mit einem Polyol ausgewählt aus der Gruppe bestehend aus 1,2-Ethandiol, 1,3-Propandiol, 2,2-Dimethyl-1,3-propandiol (Neopentandiol), 1,4-Butandiol, 2,2-Dimethyl-1,4-butanediol, 1,5-Pentandiol, 2,2-Dimethyl-1,4-butanediol, 1,6-Hexandiol oder einer Mischung aus mindestens zwei hiervon.

Bevorzugt liegt in der Frisur-stabilisierenden Zusammensetzung die Gesamtmenge an Carbodiimiden und die Gesamtmenge an Filmbildnern in einem Verhältnis von 2:1 bis 1:4, oder bevorzugt in einem Verhältnis von 1,5:1 bis 1:3, oder bevorzugt in einem Verhältnis von 1:1 bis 1:3 vor. Bevorzugt weist die Frisur-stabilisierenden Zusammensetzung mehr Gewichtsprozent an Filmbildner als an Verbindungen enthaltend mindestens drei Carbodiimidgruppen, auch Polycarbodiimid genannt.

Bevorzugt liegt die Frisur-stabilisierende Zusammensetzung in Form ausgewählt aus der Gruppe bestehend aus einem Spray, insbesondere einem Pumpsprays, einem Aerosols, einem Gel, einem Schaum, einem Schaumfestiger, einer Lotion, einem Wachs, insbesondere einem Haarwachs, einer Pomade, einem Öl, einer Milch, eine Öl in Wasser

Emulsion, einer wässrigen Lösung oder einer Creme vor. Die Frisur-stabilisierende Zusammensetzung liegt bevorzugt in Form eines Pumpsprays, eines Aerosols, eines Gels, eines Schaumes, eines Schaumfestiger, einer Lotion, eines Wachs, einer Pomade, vor.

Die erfindungsgemäße Zusammensetzung kann ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur zwischen 30°C und 200°C eine reversible Zustandsänderung fest/flüssig zeigt. Oberhalb des Schmelzpunktes wird das Wachs niedrigviskos und mischbar mit Ölen.

Der Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Schellack, Mikrowachse, Ceresin, Ozokerit, Ouricuriwachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (lkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die Wachse können in Mengen von 0 bis 10 Gew.-%, bezogen auf das Gesamtgewicht der Zusammensetzung und vorzugsweise 0 bis 5 Gew.-% enthalten sein.

Das kosmetische akzeptable Medium der erfindungsgemäßen Zusammensetzung enthält bevorzugt Wasser und gegebenenfalls ein kosmetisch verträgliches in Wasser mischbares geeignetes organisches Lösungsmittel.

Das verwendete Wasser in der erfindungsgemäßen Zusammensetzung kann ein Blütenwasser, reines demineralisiertes Wasser, Mineralwasser, Thermalwasser und/oder Meerwasser sein.

Im Fall einer O/W Zusammensetzung als erfindungsgemäße Zusammensetzung kann der Wasseranteil im Bereich von 40 bis 95 Gew.-%, bevorzugt im Bereich von 50 bis 90 Gew.-%, ganz bevorzugt im Bereich von 60 bis 80 Gew.-% bezogen auf das Gesamtgewicht der Zusammensetzung, liegen. Im Fall einer W/O Zusammensetzung liegt der Wasseranteil im Bereich von 0 bis 60 Gew.-%, bevorzugt im Bereich von 10 bis 50 Gew.-%, ganz bevorzugt im Bereich von 30 bis 50 Gew.-% bezogen auf das Gesamtgewicht der Zusammensetzung.

Die Zusammensetzung kann ferner mit einem Treibgase aufgeschäumt werden. Die oben geschriebenen Emulsionen können durch O/W-, W/O oder W/Si-Emulgator, Verdicker (wie beispielweise Hydrodispersion) oder Feststoffe (wie beispielweise Pickeringemulsion) stabilisiert sein.

Die Zusammensetzung kann einen oder mehrere Emulgatoren bzw. oberflächenaktive Mittel enthalten.

So enthalten insbesondere Öl-in-Wasser-Emulsionen (O/W) bevorzugt mindestens einen Emulgator mit einem HLB-Wert > 7 und gegebenenfalls einen Coemulgator.

Vorteilhaft werden die folgenden nichtionischen Emulgatoren eingesetzt:

- a) Partialfettsäureester und Fettsäureester mehrwertiger Alkohole und deren ethoxylierte Derivate (z. B. Glycerylmonostearat, Sorbitanstearat, Glyceryl-Stearyl-Citrat, Sucrose-Stearat)
- b) ethoxylierte Fettalkohole und Fettsäuren.

Besonders vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12, sowie Ester aus Mono-, Oligo- oder Polysacchariden mit Fettsäuren, bevorzugt Cetearylglucosid, Methylglucosedistearat.

Vorteilhafte anionische Emulgatoren sind Seifen (z. B. Natrium- oder Triethanolamin-Salze der Stearin- oder Palmitinsäure) sowie Ester der Zitronensäure wie Glycerylstearatcitrat.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemässen Zubereitungen weiter erhöht werden. Geeignete Emulgatoren sind z. B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Ricinusoel, Polyglyceryl-4-isostearat, Acrylat/$C_{10-30}$-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat.

Bei der Komponente F) handelt es sich bevorzugt um Verbindungen, die genau eine isocyanatreaktive Gruppe aufweisen, oder um Verbindungen, die mehr als eine isocyanatreaktive Gruppe aufweisen, wobei nur eine der isocyanatreaktiven Gruppen unter den gewählten Umsetzungsbedingungen mit den in der Reaktionsmischung vorhandenen Isocyanatgruppen reagiert.

Bei den isocyanatreaktiven Gruppen der Komponente F) kann es sich dabei um jede funktionelle Gruppe handeln, die mit einer Isocyanatgruppe reagieren kann, wie beispielsweise Hydroxygruppen, Thiolgruppen oder primäre und sekundäre Aminogruppen.

Isocyanatreaktive Gruppen im Sinne der Erfindung sind dabei insbesondere bevorzugt primäre oder sekundäre Aminogruppen, die mit Isocyanatgruppen unter Bildung von Harnstoffgruppen reagieren. Neben der Aminogruppe können Verbindungen der Komponente F) auch andere prinzipiell isocyanatreaktive Gruppen wie beispielsweise auch OH-Gruppen aufweisen, wobei nur eine der isocyanatreaktiven Gruppen unter den gewählten Umsetzungsbedingungen mit den in der Reaktionsmischung vorhandenen Isocyanatgruppen reagiert. Dies kann beispielsweise durch Reaktion entsprechender Aminoalkohole bei relativ niedrigen Temperaturen erfolgen, beispielsweise bei 0 bis 60°C, bevorzugt bei 20 bis 40°C. Bevorzugt wird dabei in Abwesenheit von Katalysatoren gearbeitet, welche die Reaktion von Isocyanatgruppen mit Alkoholgruppen katalysieren würden.

Beispiele für geeignete Verbindungen der Komponente F) sind primäre/sekundäre Amine, wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, Diethanolamin, 3-Amino-1-methylaminopropan, 3-Amino-1-ethylaminopropan, 3-Amino-1-cyclohexylaminopropan, 3-Amino-1-methylaminobutan, 6-Aminohexansäure, Alanin, Asparaginsäure, Glutaminsäure, Glutamin, Glycin, Ethanolamin, 3-Aminopropanol, Neopentanolamin oder Mischungen aus mindestens zwei hiervon.

Geeignete monofunktionellen Verbindungen sind auch Ethanol, n-Butanol, Ethylenglykolmonobutylether, Diethylenglykolmonomethylether, Diethylenglykolmonobutylether, Propylenglykolmonomethylether, Dipropylenglykol-monomethylether, Tripropylenglykolmonomethylether, Dipropylenglykolmono-propylether, Propylenglykolmonobutylether, Dipropylenglykolmonobutylether, Tripropylenglykolmonobutylether, 2-Ethylhexanol, 1-Octanol, 1-Dodecanol, 1-Hexadecanol.

Bevorzugt wird zur Herstellung des bevorzugt amorphen Polyurethanharnstoffs $\geq$ 0,1 und $\leq$ 20 Gew.-% der Komponente F) und besonders bevorzugt $\geq$ 0,3 und $\leq$ 10 Gew.-% der Komponente G), jeweils bezogen auf die Gesamtmasse des bevorzugt amorphen Polyurethanharnstoffs, eingesetzt.

Bevorzugt wird die Komponente G) eingesetzt und das molare Verhältnis von Komponente F) zu Komponente G) beträgt bevorzugt 5:1 bis 1:5, besonders bevorzugt 1,5:1 bis 1:4 und ganz besonders bevorzugt 1:1 bis 1:3.

Bevorzugt werden zur Herstellung des bevorzugt amorphen Polyurethanharnstoffes die Komponenten A) bis G) in den folgenden Mengen eingesetzt, wobei sich die Einzelmengen stets zu 100 Gew.-% addieren:

5 bis 40 Gew.-% Komponente A),

55 bis 90 Gew.-% Komponente B),

0,5 bis 20 Gew.-% Summe der Komponenten C1) und gegebenenfalls E),

0,1 bis 10 Gew.-% Komponente C2),

0 bis 20 Gew.-% Komponente D),

0,1 bis 20 Gew.-% Komponente F) und

0 bis 10 Gew.-% Komponente G).

Bevorzugt werden zur Herstellung des bevorzugt amorphen Polyurethanharnstoffes die Komponenten A) bis G) in den folgenden Mengen eingesetzt, wobei sich die Einzelmengen stets zu 100 Gew.-% aufaddieren:

10 bis 35 Gew.-% Komponente A),

60 bis 85 Gew.-% Komponente B),

1 bis 15 Gew.-% Summe der Komponenten C1) und gegebenenfalls E),

0,5 bis 4 Gew.-% Komponente C2),

0 bis 10 Gew.-% Komponente D),

0,3 bis 10 Gew.-% Komponente F) und

0,1 bis 3 Gew.-% Komponente G).

Bevorzugt umfasst die Frisur-stabilisierende Zusammensetzung einen Polyurethanharnstoff, der erhältlich ist, durch Umsetzung von mindestens

A) einer bevorzugt aliphatischen Polyisocyanat-Komponente mit einer mittleren Isocyanatfunktionalität von ≥ 1,8 und ≤ 2,6, welche ausgewählt ist aus HDI, IPDI und/oder H12-MDI oder deren Modifikationsprodukten,

B) einer polymeren Polyol-Komponente, welche bevorzugt Carboxy- oder Carboxylatgruppen enthalten,

C) einer aminofunktionellen Kettenverlängerer-Komponente mit mindestens 2 isocyanatreaktiven primären und/oder sekundären Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,

D) gegebenenfalls weiteren hydrophilierenden Komponenten, die unterschiedlich sind von C2),

E) gegebenenfalls hydroxyfunktionellen Verbindungen mit einem Molekulargewicht von 62 bis 399 mol/g,

F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und

G) gegebenenfalls einer aliphatischen Polyisocyanat-Komponente mit einer mittleren Isocyanatfunktionalität von > 2,6 und ≤ 4, wobei die Komponente G) aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur besteht.

Bevorzugt umfasst die Frisur-stabilisierende Zusammensetzung einen bevorzugt amorphen Polyurethanharnstoff, der erhältlich ist, durch Umsetzung von mindestens

A) einer bevorzugt aliphatischen Polyisocyanat-Komponente, wobei es sich um ein Gemisch aus IPDI und HDI handelt,

B) einer polymeren Polyether-Polyol-Komponente, welche ein Gemisch aus wenigstens zwei Poly(propylenglykol)polyetherpolyole ist und wobei sich die Poly(propylenglykol)polyetherpolyole in ihren zahlenmittleren Molekulargewichten unterscheiden,

C) einer aminofunktionellen Kettenverlängerer-Komponente mit 2 isocyanatreaktiven primären und/oder sekundären Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,

D) gegebenenfalls weiteren hydrophilierenden Komponenten, die unterschiedlich sind von C2), wobei es sich um nichtionisch hydrophilierende Komponenten D1) handelt,

E) gegebenenfalls hydroxyfunktionellen Verbindungen mit einem Molekulargewicht von 62 bis 399 mol/g,

F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert, wobei es sich bei der isocyanat reaktiven Gruppe um eine primäre und/oder sekundäre Amino- und/oder Hydroxygruppe handelt und

G) gegebenenfalls einer aliphatischen Polyisocyanat-Komponente mit einer mittleren Isocyanatfunktionalität von > 2,6 und ≤ 4, wobei die Komponente G) aus einem aliphatischen oder cycloaliphatischen Polyisocyanat-Oligomer mit Isocyanurat-, Urethan-, Allophanat-, Biuret-, Iminooxadiazindion- oder Oxadiazintrionstruktur, basierend auf HDI, IPDI und/oder H12-MDI besteht.

Bevorzugt ist die Frisur-stabilisierenden Zusammensetzung zur Behandlung von Haaren, insbesondere zur Strukturerhaltung von Farb- und/oder Formveränderung der Haare, geeignet. Insbesondere wird bei der Behandlung der Haare mit der Frisur-stabilisierenden Zusammensetzung eine weitere haarkosmetische Anwendung vorgenommen. Die weitere haarkosmetische Anwendung kann jede Anwendung an dem Haar sein, die zu einer Farb- und/oder Formveränderung des Haares führt. Durch die Behandlung des Haares mit der erfindungsgemäßen Zusammensetzung wird bevorzugt erreicht, dass das Haar eine höhere Strukturbeständigkeit gegenüber Wasser z.B. in Form eines Wasserstrahls z.B. beim Duschen, Baden oder Schwimmen, oder Haarpflegemitteln wie Shampoo, Haarfestiger etc. erhält, eine geringer Quellung und eine gute Haptik aufweist. Bevorzugt ist die haarkosmetische Anwendung ausgewählt aus der Gruppe bestehend aus

(A) einem Färben mindestens eines Teils der Haare,
(B) einer Dauerwellenbehandlung der Haare,
(C) einer Behandlung mit einem Lockenstab,
(D) einem Glätten der Haare,
(E) einer dauerhaften Glättung der Haare,
(F) einem Frisieren der Haare,
(G) eine Haarverlängerung,
(H) einer Haarverkürzung,
(I) einer Kombination aus mindestens zwei Anwendungen ausgewählt aus (A) bis (G).

Die Behandlung von Haaren mit der erfindungsgemäßen Frisur-stabilisierenden Zusammensetzung findet bevorzugt unter Anwendung von erhöhter Temperatur statt. Die Anwendung von erhöhter Temperatur kann sowohl die zu behandelnden Haare als auch die zu verwendende Frisur-stabilisierende Zusammensetzung oder beides betreffen. Bevorzugt findet die Anwendung von erhöhter Temperatur ausgewählt aus der Gruppe bestehend aus Erwärmung der Frisur-stabilisierenden Zusammensetzung vor Anwendung auf dem Haar, Erwärmung der Haare vor Behandlung mit der Frisur-stabilisierenden Zusammensetzung, Erwärmung der Haare nach Behandlung mit der Frisur-stabilisierenden Zusammensetzung, Erwärmung der Haare während der Behandlung mit der Frisur-stabilisierenden Zusammensetzung oder einer Kombination aus mindestens zwei hieraus statt. Bevorzugt findet die Anwendung von erhöhter Temperatur durch Erwärmen der Frisur-stabilisierenden Zusammensetzung oder Erwärmen des Haares vor Behandlung der Haare mit der Frisur-stabilisierenden Zusammensetzung statt. Die Anwendung von erhöhter Temperatur findet wahlweise vor, während und/oder nach der Behandlung von Haaren mit der erfindungsgemäßen Frisur-stabilisierenden Zusammensetzung statt. Bevorzugt wird das zu behandelnde Haar bei der Anwendung von erhöhter Temperatur mit einem Mittel in Kontakt gebracht, das eine Temperatur in einem Bereich von 30 bis 250 °C, oder bevorzugt in einem Bereich von 40 bis 230 °C, oder bevorzugt in einem Bereich von 50 bis 200 °C, oder bevorzugt in einem Bereich von 60 bis 180 °C aufweist. Bevorzugt findet das Erwärmen der Frisur-stabilisierenden Zusammensetzung vor Behandlung der Haare damit statt, bevorzugt in einem Bereich von 30 bis 100 °C, oder bevorzugt in einem Bereich von 35 bis 90 °C, oder bevorzugt in einem Bereich von 40 bis 80 °C statt. Bevorzugt wird die erhöhte Temperatur dem Haar über erwärmte Luft oder eine erwärmte Oberfläche zugeführt. Die Erhöhung der Temperatur wird bevorzugt durch ein Mittel erreicht ausgewählt aus der Gruppe bestehend aus einem Föhn, einem Glätteisen, einem Lockeneisen, einer Trockenhaube, einem Lockenstab oder einer Kombination aus mindestens zwei hiervon. Die angegebenen Temperaturbereiche für die erhöhte Temperatur ist dabei bevorzugt die Temperatur des Mittels unmittelbar bevor es mit dem Haar in Kontakt gebracht wird. Das Erwärmen der Frisur-stabilisierenden Zusammensetzung kann auch durch das Erwärmen des Haares vor der Behandlung mit der Frisur-stabilisierenden Zusammensetzung stattfinden, insbesondere dann, wenn das Haar mit einem Mittel behandelt wird, dass eine Temperatur von mehr als 100 °C aufweist.

Die Dauer der Anwendung von erhöhter Temperatur liegt bevorzugt in einem Bereich von 1 Sekunde bis 3 Stunden, oder bevorzugt in einem Bereich von 5 Sekunden bis 2 Stunden, oder bevorzugt in einem Bereich von 10 Sekunden bis 1 Stunde, oder bevorzugt in einem Bereich von 20 Sekunden bis 50 Minuten. Je nach Art der Gestaltung der Frisur, zum Beispiel Glättung des Haares oder Lockung des Haares, kann die Anwendung der erhöhten Temperatur unterschiedlich lange gestaltet werden und bei unterschiedlich hohen Temperaturen erfolgen. So ist es bevorzugt bei

der Glättung des Haares eine Temperatur über 200 °C mehrmals, bevorzugt 2 bis 10 mal für wenige Sekunden, bevorzugt 2 bis 30 Sekunden, oder bevorzugt 3 bis 10 Sekunden anzuwenden. Dahingegen ist es bevorzugt die Temperatur beim Gestalten von Locken in einem Bereich von 50 bis 100 °C zu halten und dafür eine Anwendung der Temperatur in einem Bereich von 10 bis 120 Minuten, oder bevorzugt in einem Bereich von 30 bis 100 Minuten vorzunehmen.

[A10] In einer bevorzugten Ausführungsform der Zusammensetzung ist die Polycarbodiimid-Komponente (V2) aus aliphatischen oder cycloaliphatischen Polyisocyanaten hergestellt.
Die aliphatischen bzw. cycloaliphatischen Polyisocyanat sind bevorzugt ausgewählt aus der Gruppe bestehend aus Methylendiisocyanat, Dimethylendiisocyanat, Trimethylendiisocyanat, Tetramethylendiisocyanat, Pentamethylendiisocyanat, Dipropyletherdiisocyanat, 2,2-Dimethylpentandiisocyanat, 3-Methoxyhexandiisocyanat, Octamethylendiisocyanat, 2,2,4-Trimethylpentandiisocyanat, Nonamethylendiisocyanat, Decamethylen-diisocyanat, 3-Butoxyhexandiisocyanat, 1,4-Butylenglykoldipropyletherdiisocyanat, Thiodihexyldiisocyanat, Metaxylylendiisocyanat, Paraxylylendiisocyanat, Tetramethylxylylendiisocyanat, 4,4'-Dicyclohexylmethandiisocyanat (H12MDI), Isophorondiisocyanat (IPDI), Hexamethylendiiso-cyanat (HDI), hydriertes Xylylendiisocyanat (H6XDI), 1,12-Diisocyanatdodecan (DDI), Nor-bornandiisocyanat (NBDI) und 2,4-Bis(8-isocyanatoctyl)-1,3-dioctylcyclobutan (OCDI) oder einer Mischung aus mindestens zwei hieraus. Besonders bevorzugt sind Isophorondiisocyanat (IPDI), Hexamethylendiisocyanat (HDI), hydriertes Xylylendiisocyanat ($H_6XDI$), 4,4'-Dicyclohexylmethandiisocyanat (H12MDI). Ganz besonders bevorzugt ist das cycloaliphatische Polyisocyanat 4,4'-Dicyclohexylmethandiisocyanat (H12MDI).
[A11] In einer bevorzugten Ausführungsform der Zusammensetzung beinhaltet die Polycarbodiimid-Komponente (V2) Polycarbodiimid-Verbindungen, enthaltend mindestens drei Carbodiimidgruppen, aufgebaut entsprechend der Formel (I),

$$R^4 - [R^1- N=C=N]_n - R^5 \qquad (I),$$

wobei

n für eine ganze Zahl von 3 bis 100, bevorzugt von 3 bis 50, oder bevorzugt von 3 bis 20, oder bevorzugt von 3 bis 10 steht,

$R^1$ und $R^5$ jeweils unabhängig voneinander für lineare oder verzweigte, aliphatische oder cyclo-aliphatische, gegebenenfalls substituierte Gruppen mit 4 bis 100, oder bevorzugt von 4 bis 50, oder bevorzugt von 4 bis 20 C-Atomen steht.

$R^4$ für lineare oder verzweigte, aliphatische oder cyclo-aliphatische, gegebenenfalls substituierte Gruppen mit 1 bis 100, oder bevorzugt von 1 bis 50, oder bevorzugt von 1 bis 20 C-Atomen steht.

Als Substituenten der Reste $R^4$ und/oder $R^5$ kommen im Rahmen der Erfindung zahlreiche organische Gruppen in Frage, beispielsweise Alkyl-, Cycloalkyl-, Aryl-, Alkoxy, Halogen-, Ether-, Thioether-, Thiourethan-, Disulfid-, Sulfoxid-, Sulfon-, Sulfonat-, Amino-, Aldehyd-, Keto-, Carbonsäureester-, Carbonsäure-, Carbonat-, Carboxylat-, Cyano-, Alkylsilan- und Alkoxysilangruppen, Urethangruppen, Allophanatgruppen, Biuretgruppen, Harnstoffgruppen sowie Carboxylamidgruppen. Insbesondere ist ein Teil, auch Substituent genannt, der Reste $R^1$, $R^4$ oder $R^5$, bevorzugt der Reste $R^4$ und/oder $R^5$ ausgewählt aus der Gruppe bestehend aus einer Polyethylenglycolgruppe, einer Polypropylengruppe, einer Methoxygruppe, einer Ethoxygruppe, einer Butoxygruppe, einer Methoxpolyethylenglycolgruppe, einer Urethangruppe, einer Alkylgruppe, einer Cycloalkylgruppe oder einer Kombination aus mindestens zwei hieraus.
Weiterhin bevorzugt weist mindestens einer der Reste $R^1$, $R^4$ oder $R^5$, bevorzugt die Reste $R^4$ und/oder $R^5$ sowohl eine Alkyl- als auch eine Cycloalkylgruppe auf. Darüber hinaus ist es bevorzugt, dass mindestens einer, besonders bevorzugt beide der Reste $R^4$ oder $R^5$, mindestens eine der Gruppen, bevorzugt mindestens zwei der Gruppen ausgewählt aus der Gruppe bestehend aus einer Urethangruppe, einer Polyethylenglycolgruppe (PEG), einer Polypropylengruppe (PPG), einer Methoxygruppe, einer Ethoxygruppe, einer Alkylgruppe und einer Cycloalkylgruppe oder einer Kombination aus mindestens zwei hiervon aufweist. Insbesondere ist es bevorzugt, dass die Reste $R^4$ und/oder $R^5$ eine Alkylgruppe, eine Cycloalkylgruppe, eine Urethangruppe, eine PEG-Gruppe und eine Methoxygruppe, bevorzugt genau in dieser Reihenfolge aufweisen. Bevorzugt enthalten beide Reste $R^4$ und/oder $R^5$ zumindest eine Urethangruppe sowie zumindest eine Gruppe, die für einen von einem $C_1$ bis $C_{30}$ Alkohol oder einem $C_1$ bis $C_{30}$ Monoalkoxyethylenglykol abgeleiteten Rest stehen.
Bei der Polycarbodiimid-Komponente, enthaltend mindestens drei Carbodiimidgruppen, im Folgenden auch Polycarbodiimid genannt, handelt es sich bevorzugt um wasserlösliche oder wasserdipergierbare Verbindungen.
Das Polycarbodiimid, das in der erfindungsgemäßen Zusammensetzung enthalten ist hat bevorzugt ein Molekulargewicht $M_w$ (bestimmt mittels GPC) in einem Bereich von 300 bis 500 000 g/mol, oder bevorzugt in einem Bereich

von 500 bis 300 000 g/mol, oder bevorzugt in einem Bereich von 1000 bis 200 000 g/mol.

Ein bevorzugtes Verfahren zur Herstellung von wässrigen Dispersionen von Polycarbodiimiden, umfasst wenigstens einen Schritt, wobei in dem wenigstens einen Schritt wenigstens ein aliphatisches oder cycloaliphatisches Polyisocyanat bei einer Temperatur im Bereich von 160 bis 230 °C in Gegenwart von Carbodiimidisierungskatalysator zu einem Polycarbodiimid mit einer mittleren Funktionalität von größer 3, bevorzugt von 3 bis 100, oder bevorzugt von 3 bis 50, oder bevorzugt von 3 bis 20, oder bevorzugt von 3 bis 10 Carbodiimideinheiten umgesetzt wird. Bevorzugt werden die Reaktionsgase zeitweise oder permanent abgeführt. Bevorzugt beträgt die Menge an Carbodiimidisierungskatalysator 50 bis 3000 ppm, bezogen auf die molare Menge an Polyisocyanat.

Im Sinne der vorliegenden Erfindung bedeutet die mittlere Funktionalität von Carbodiimideinheiten die durchschnittliche Anzahl der Carbodiimideinheiten. Die mittlere Funktionalität kann auch eine gebrochene Zahl sein. Die mittlere Funktionalität beträgt bevorzugt 3 bis 50, oder bevorzugt 3 bis 20, oder bevorzugt 3 bis 10. Je höher die Funktionalität ist, desto niedriger ist die Dispergierbarkeit des hydrophilierten Polycarbodiimids in Wasser.

Bevorzugt ist der Carbodiimidisierungskatalysator eine Organophosphorverbindung, besonders bevorzugt Organophosphorverbindungen ausgewählt aus der Gruppe bestehend aus Phos-phanoxid, Phospholanoxid und Phospholenoxid, sowie deren Sulfo- und Iminoanaloga. Die Phosphan-, Phospholen- und Phospholan-Oxide, -Sulfide sowie -Iminoderivate können unter anderem aus entsprechenden Vorgängern mit dreiwertigem Phosphor wie Phosphanen, Phospholanen und Phospholenen in situ generiert werden. Bevorzugt wird das Phospholenoxid aus der Gruppe bestehend aus 3-Methyl-1-phenyl-2-phospholen-1-oxid, 3-Methyl-1-ethyl-2-phospholen-1-oxid, 1,3-Dimethyl-2-phospholen-l-oxid, 1-Phenyl-2-phospholen-1-oxid, 1-Ethyl-2-phospholen-1-oxid, 1-Methyl-2-phospholen-1-oxid ausgewählt. Weitere geeignete Katalysatoren sowie bevorzugte Ausführungsformen des Verfahrens für die Herstellung des Polycarbodiimids sind in der WO 2011/120928 A2 beschrieben

Ein bevorzugtes Verfahren zur Herstellung von wässrigen Dispersionen von Polycarbodiimid umfasst die Schritte:

a) Umsetzen von wenigstens einem aliphatischem oder cycloaliphatischem Polyisocyanat bei einer Temperatur im Bereich von 160 bis 230 °C in Gegenwart von 50 bis 3000 ppm (ppm = part per million, 1 ppm = 0.0001%, molarer Anteile) Carbodiimidisierungskatalysator bezogen auf die molare Menge an Polyisocyanat zu einem Polycarbodiimid, bevorzugt mit einer mittleren Funktionalität von 3 bis 20 Carbodiimideinheiten umgesetzt wird, wobei die Reaktionsgase zeitweise oder permanent gezielt aus dem Reaktionsmedium abgeführt werden,

b) Umsetzen des in Schritt a) erhaltenen Polycarbodiimids mit wenigstens einer hydrophilen Verbindung, die mindestens gegenüber Isocyanat- und/oder Carbodiimidgruppen reaktionsfähige Gruppe trägt, zum Beispiel aber nicht darauf beschränkt, ausgewählt aus der Gruppe bestehend aus Polyethoxymonoolen, Polyethoxydiolen, Polyethoxypolypropoxy-monoolen, Polyethoxypolypropoxydiolen, Polyethoxymonoaminen, Polyethoxydiaminen, Polyethoxypolypropoxymonoaminen, Polyethoxypolypropoxydiaminen, Hydroxyalkylsulfonaten, Aminalkylsulfonaten, Polyethoxymono- und -dithiolen, Polyethoxymono- und - dicarbonsäuren, Mono- und Dihydroxycarbonsäuren bzw. deren Salzen zu einem hydrophilierten Carbodiimid, gegebenenfalls weiteres Umsetzen der nicht abreagierten Isocyanatgruppen mit weiteren gegenüber Isocyanatgruppen reaktionsfähigen Verbindungen, wie zum Beispiel mit Wasser, Alkoholen, Thiolen, Aminen, Mineral- und Carbonsäuren und

c) Dispergieren der in Schritt b) erhaltenen Verbindung in Wasser.

Bevorzugt erfolgt die Umsetzung des in Schritt a) erhaltenen, Isocyanat-Gruppen enthaltenden Polycarbodiimids gemäß der vorliegenden Erfindung so, dass 10 bis 70 Molprozent (mol.-%) der im Polycarbodiimid vorhandenen Isocyanat-Gruppen mit mindestens einer hydrophilen Verbindung als Teilschritt b1) des Schritts b) umgesetzt werden, wobei die hydrophile Verbindung ausgewählt ist aus der Gruppe bestehend aus Polyethoxymonoolen, Polyethoxydiolen, Polyethoxypolypropoxymonoolen, Polyethoxypolypropoxydiolen, Polyethoxymono-aminen, Polyethoxydiaminen, Polyethoxypolypropoxymonoaminen, Polyethoxypoly-propoxydiaminen, Hydroxyalkylsulfonaten, Aminalkylsulfonaten, Polyethoxymono- und -dithiolen, Polyethoxymono- und -dicarbonsäuren. Im Teilschritt b2) des Schritts b) werden 30 bis 90 mol.-% der verbleibenden Isocyanat-Gruppen dann mit mindestens einer gegenüber Isocyanatgruppen reaktionsfähigen Verbindung umgesetzt, zum Beispiel, Polyethoxymonoolen, Polyethoxydiolen, Polyethoxypolypropoxymonoolen, Polyethoxypolypropoxydiolen, Polyethoxymonoaminen, Polyethoxydiaminen, Polyethoxypolypropoxymonoaminen, Polyethoxypolypropoxydiaminen, Hydroxyalkylsulfonaten, Aminalkylsulfonaten, Polyethoxymono- und -dithiolen, Polyethoxy-mono- und -dicarbonsäuren, Wasser, $C_1$ bis $C_{30}$ Alkoholen, $C_1$ bis $C_{30}$ Thiolen, Aminen, Mi-neral- und Carbonsäuren.

Bevorzugt erfolgt die Umsetzung der Polycarbodiimide in Teilschritt b1) des Schritts b) mit wenigstens einer Verbindung ausgewählt aus der Gruppe der Verbindungen entsprechend der allgemeinen Formel (III):

$$R^6 - O - (CH_2 - CH_2 - O)_m - H \qquad (III)$$

mit $R^6 = C_1$ bis $C_{30}$ Alkyl oder Acylgruppe und m = 4 bis 30

besonders bevorzugt mit wenigstens einer Verbindung ausgewählt aus der Gruppe entsprechend der Formel (III), wobei $R^6$ eine Methylgruppe ist und m = 10 bis 30.

Ganz besonders bevorzugt ist Monomethoxypolyethylenglykol mit m = 15-20.

Bevorzugte C1 bis C30 Alkohole, die zu einer Weiterreaktion der im Polycarbodiimid vorhandenen und mit den hydrophilen Verbindungen nicht vollständig umgesetzten Isocyanatgruppen in Teilschritt b2) des Schritts b) verwendet werden können, sind zum einen Wasser, niedermolekulare Monoalkohole oder auch Diole mit einem Molekulargewicht bevorzugt von 32 bis 500, besonders bevorzugt von 62 bis 300 g/mol. Ganz besonders bevorzugt werden kurzkettige Monoalkohole also verzweigte oder unverzweigte Monoalkohole mit 1 bis 30 C-Atomen wie Methanol, Ethanol, Propanol, 1-Butanol, 1-Pentanol, 1-Hexanol, Cyclohexanol, Cyclohexylmethanol, 2-Ethylhexanol, Dodecanol, Stearylalkohol oder Oleylalkohol, deren Gemische untereinander und Gemische deren Isomere sowie kurzkettige Dialkohole mit 2 bis 60 C-Atomen wie 1,4-Butandiol, 1,5-Pentandiol, 1,6-Hexandiol, Cyclohexandiol, Cyclohexandimethanol, 1,8-Octandiol, 1,9-Nonadiol, 1,10-Decandiol, 1,11-Undecandiol, 1,12-Dodecandiol, 1,13-Tridecandiol, Tricyclodecandimethanol, deren Gemisch untereinander sowie Gemische deren Isomere verwendet.

Die Reihenfolge der Teilschritte b1) und b2) des Schritts b) kann so festgelegt sein, dass Teil-schritt b1) vor Teilschritt b2), beide gleichzeitig oder in der umgekehrten Reihenfolge erfolgen kann.

Optional wird die wässrige Dispersion von hydrophiliertem Polycarbodiimid in einem Schritt d) auf einen pH-Wert im Bereich von 7 bis 12 (bei 23°C), besonders bevorzugt im Bereich von 8 bis 11, eingestellt. Dafür können Aminlösungen, Laugen und herkömmliche Pufferlösungen verwendet werden.

Zusammensetzung weist die Verbindung, enthaltend mindestens drei Carbodiimidgruppen, die allgemeine Formal (II) auf:

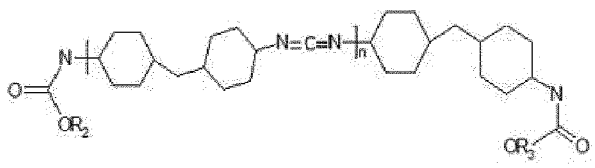

(II)

worin $R_2$ und $R_3$, unabhängig voneinander, für einen von einer Verbindung ausgewählt aus der Gruppe bestehend aus einem Monoalkoxy-poly(ethylenglykol) gemäß der allgemeinen Formel (III)

$$R^6 - O - (CH_2 - CH_2 - O)_m - H \qquad (III)$$

mit $R^6 = C_1$ bis $C_{30}$ Alkyl oder Acylgruppe und m = 4 bis 30

und einem von $C_1$ bis $C_{30}$ Alkohol oder einem $C_1$ bis $C_{30}$ Monoalkoxyethylenglykol abgeleiteten Rest stehen.

Die wässrigen Polycarbodiimid-Dispersionen und/oder Lösungen, hergestellt nach dem oben beschriebenen Verfahren, haben üblicherweise einen Feststoffgehalt von 10 bis 80 Gew.%, bevorzugt von 20 bis 60 Gew.-% und besonders bevorzugt von 30 bis 50 Gew.%.

Bevorzugt enthält die Frisur-stabilisierende Zusammensetzung die Verbindung enthaltend das Carbodiimid, auch Polycarbodiimid (V2) genannt, der Formel (I) oder (III) in einem Bereich von 0,01 bis 50 Gew.-%, bevorzugt in einem Bereich von 0,05 bis 30 Gew-%, oder bevorzugt in einem Bereich von 0,1 bis 20 Gew.-%, oder bevorzugt in einem Bereich von 0,5 bis 10 Gew.-%, bezogen auf das Gesamtgewicht der Frisur-stabilisierenden Zusammensetzung. Je nach Anwendung der Frisur-stabilisierenden Zusammensetzung beispielsweise in Haaranwendungsprodukten, können die bevorzugten Mengen an Polycarbodiimiden stark variieren. Als Haaranwendungsprodukte werden Produkte wie Haarstyling-, Haarpflege- oder Haarfärbeprodukte verstanden. Darüber hinaus kann die Frisur-stabilisierende Zusammensetzung auch kolorierende Substanzen beinhalten und eine Wimperntusche, ein Mascara, ein Shampoo, ein Festiger, ein Färbemittel, wobei dies jeweils als Spray, Lotion, Creme, Schaums, Lösung, Emulsion oder Wachs vorliegen kann, sein oder Bestandteil von einem solchen sein. Die Frisur-stabilisierende Zusammensetzung enthält das Polycarbodiimid bevorzugt in einem Bereich von 0,1 bis 10 Gew.-%, oder bevorzugt in einem Bereich von 0,2 bis 8 Gew.-%, oder bevorzugt in einem Bereich von 0,5 bis 5 Gew.-%, bezogen auf das Gesamtgewicht der Frisur-stabilisierenden Zusammensetzung, beispielsweise in Shampoos. Die Frisur-stabilisierende Zusammensetzung enthält das Polycarbodiimid bevorzugt in einem Bereich von 0,5 bis 30 Gew.-%, oder bevorzugt in einem Bereich von 1 bis 20 Gew.-%, oder bevorzugt in einem Bereich von 2 bis 15 Gew.-%, bezogen auf das Gesamtgewicht der Frisur-stabilisierenden Zusammensetzung, in Haarfärbeprodukten.

Alle im Zusammenhang mit der erfindungsgemäßen Frisur-stabilisierenden Zusammensetzung erwähnte und beschriebene Komponenten, deren bevorzugte Mengen sowie deren Eigenschaften sind frei kombinier- und auswählbar.

[A12] Ein weitere Gegenstand der Erfindung betrifft eine Verwendung mindestens eines Polyurethanharnstoffes (V1), gegebenenfalls zusammen mit einem Polycarbodiimid (V2), zur Herstellung einer Frisur-stabilisierenden Zusammensetzung die geeignet ist Frisuren wasserstabil zu generieren, wobei der Polyurethanharnstoff (V1) erhältlich ist durch Umsetzung von wenigstens

A) einer Polyisocyanat-Komponente mit einer mittleren Isocyanatfunktionalität von $\geq 1,8$ und $\leq 2,6$,

B) einer polymeren Polyol-Komponente,

C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionellen Verbindung C2), die ionische oder ionogene Gruppen aufweist,

D) gegebenenfalls weiteren hydrophilierenden Komponenten, die unterschiedlich sind von C2),

E) gegebenenfalls hydroxyfunktionellen Verbindungen mit einem Molekulargewicht von 62 bis 399 mol/g,

F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und

G) gegebenenfalls einer aliphatischen Polyisocyanat-Komponente mit einer mittleren Isocyanatfunktionalität von $> 2,6$ und $\leq 4$,

wobei der Polyurethanharnstoff mindestens eine der Komponenten C2) oder D) aufweist, und wobei der Polyurethanharnstoff Carboxygruppen oder Carboxylatgruppen aufweist.
Was unter einer wasserstabilen Frisur zu verstehen ist wurde bereits für die erfindungsgemäße Frisur-stabilisierende Zusammensetzung beschrieben. Alle für die Frisur-stabilisierende Zusammensetzung erwähnten Eigenschaften, Bestandteile von Komponenten, bevorzugte Anteile von Komponenten, insbesondere der Komponenten (V1), A) bis G) sowie (V2) gelten ebenfalls für die Verwendung des Polyurethanharnstoffs (V1), gegebenenfalls mit dem Polycarbodiimid (V2) wie für die erfindungsgemäße Frisur-stabilisierende Zusammensetzung beschrieben.

[A13] Ein weiterer Gegenstand der Erfindung ist die Verwendung der erfindungsgemäßen Frisur-stabilisierenden Zusammensetzung zur Verbesserung der Wasserbeständigkeit von erzeugten Frisuren. Auch hier wird für die Beschreibung und Definition der Wasserbeständigkeit auf die Beschreibung von Frisuren, deren Herstellung und Eigenschaften im Zusammenhang mit der erfindungsgemäßen Frisur-stabilisierenden Zusammensetzung verwiesen.

[A14] Ein weiterer Gegenstand der Erfindung betrifft ein Verfahren zur Gestaltung einer Frisur, insbesondere zum wasserbeständigen und/oder waschbeständigen Formen von Haaren zu einer Frisur, mit mindestens den Schritten:

F1) Behandeln des Haares eines Anwenders mit einer Frisur-stabilisierenden Zusammensetzung;

F2) Gegebenenfalls Wärmebehandeln des Haares des Anwenders, wobei Schritt F2) vor und/oder nach Schritt F1) erfolgen kann;

F3) Formen des Haares zu einer Frisur;

F4) Gegebenenfalls Trocknen des Haares;

F5) Gegebenenfalls in Kontaktbringen des Haares mit Wasser vor während oder nach einem der Schritte F1) bis F4), bevorzugt vor Schritt F1);

F6) gegebenenfalls mindestens einmaliges Wiederholen mindestens eines Teils der Schritte F4) und F5).

Unter Kontaktbringen des Haares mit Wasser unter Punkt F5) wird erfindungsgemäß verstanden, dass mindestens ein Teil des in Schritt F3) in Form gebrachten Haares in Wasser eingetaucht wird. Was unter Wasser zu verstehen ist, wurde bereits oben definiert. Hierbei ist bevorzugt ein Waschvorgang mit tensidhaltigem Wasser, auch shampoonieren genannt, in dem in Kontaktbringen der Haare mit eingeschlossen. Das Behandeln in Schritt F1) mit der erfindungsgemäßen Zusammensetzung kann dabei in einem oder in zwei Schritten erfolgen. Erfolgt das Behandeln in Schritt F1) in zwei Schritten, so wird zunächst das Polyurethanharnstoff (V1) als einzelne Komponente auf das Haar gebracht und in einem anschließenden Schritt die Polycarbodiimid-Komponente (V2) oder umgekehrt. Wird das Behandeln in Schritt F1) in einem Schritt durchgeführt, so liegen die beiden Komponenten (V1) und (V2) bereits gemischt vor. Bevorzugt wird Schritt F5) vor Schritt F1) vorgenommen, die erfindungsgemäße Zusammensetzung also in das nasse Haar eingebracht.
Bevorzugt wird zur Gestaltung der Frisur zunächst Schritt F5) vorgenommen, anschließend Schritt Fl), danach Schritt F3) und anschließend ggf. Schritt F2) oder F4). Bevorzugt wird Schritt F3) gemeinsam mit Schritt F3) ausgeführt, insbesondere dann, wenn das Haar direkt mit dem Mittel zur Gestaltung des Haares beheizt ist. Vorteilhafterweise kann die Frisur nun über mehrere Schritte F5) insbesondere in Form von Shampoonieren wiederholt werden, ohne das die Frisur gänzlich neu gestaltet werden muss. Bevorzugt bleibt die Frisur nach jedem Schritt F5) zu mindestens 50 %, oder bevorzugt zu mindestens 70 %, oder zu mindestens 90 % im Vergleich zum Zustand vor dem vorherigen Schritt F5) bestehen.

[A15] Ein weiterer Gegenstand der Erfindung betrifft ein Verfahren zur Herstellung einer Frisur-stabilisierenden Zusammensetzung mindestens mit den Schritten:

i. Herstellen eines Polyurethanharnstoff (V1) aus einem isocyanatgruppenhaltigen Prepolymer a., welches erhältlich ist aus der Reaktion von mindestens den Komponenten

A) mindestens einem Polyisocyanat mit einer Funktionalität von ≥ 1,5 bis ≤ 6, bevorzugt von ≥ 1,5 bis ≤ 3; mit

B) mindestens einem Polyol mit einer Funktionalität von ≥ 1,5 bis ≤ 6, bevorzugt von ≥ 1,5 bis ≤ 3;

ii. Umsetzen des Prepolymer a. mit mindestens

C) einer Mischung C) aus primären und/oder sekundären aminofunktionellen Verbindungen C1) und aus primären und/oder sekundären Diaminoverbindungen C2) zu dem Polyurethanharnstoff (V1);

iii. Mischen des Polyurethanharnstoff (V1) mit mindestens einem Polycarbodiimid (V2) unter Erhalt der Frisur-stabilisierenden Zusammensetzung.

Bevorzugt handelt es sich bei dem Polyurethanharnstoff (V1) um den im Zusammenhang mit der erfindungsgemäßen Frisur-stabilisierenden Zusammensetzung beschriebenen Polyurethanharnstoff (V1). Bevorzugt handelt es sich bei dem Polycarbodiimid (V2) um das im Zusammenhang mit der erfindungsgemäßen Frisur-stabilisierenden Zusammensetzung beschriebenen Polycarbodiimid (V2). Bevorzugt sind sämtliche Komponenten, deren Eigenschaften, deren bevorzugte Mengen davon in allen erwähnten Kombinationen ebenfalls für die gleichnamigen Komponenten im Zusammenhang mit dem erfindungsgemäßen Verfahren zur Herstellung einer Frisur-stabilisierenden Zusammensetzung übertragbar. Auch für die Auswahl und Herstellung der einzelnen Komponenten unter Schritt i. bis iii. wird auf die Beschreibung der erfindungsgemäßen Frisur-stabilisierenden Zusammensetzung verwiesen. Die Bedingungen unter denen Komponente A) mit B) in Schritt i. reagiert sind dem Fachmann hinlänglich bekannt. Bevorzugt wird die Reaktion der Komponnten A) und B) bei einer Temperatur in einem Bereich von 30 bis 100 °C, bevorzugt in einem Bereich von 40 bis 90 °C durchgeführt. Die Komponenten D) bis F) werden, wenn überhaupt eingesetzt, bevorzugt zusammen mit der Komponente C) in Schritt ii. eingesetzt. Komponente G) wird, wenn überhaupt eingesetzt, bevorzugt mit den Komponenten A) und B) in Schritt i. eingesetzt.
Ein weiterer Gegenstand der Erfindung betrifft ein Kit, aufweisend mindestens die Komponenten (V1) und (V2), insbesondere

(V1) einen Polyurethanharnstoff, welcher erhältlich ist durch Umsetzung von wenigstens

A) einer Polyisocyanat-Komponente mit einer mittleren Isocyanatfunktionalität von ≥ 1,8 und ≤ 2,6,

B) einer polymeren Polyol-Komponente,

C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,

D) gegebenenfalls weiteren hydrophilierenden Komponenten, die unterschiedlich sind von C2),

E) gegebenenfalls hydroxyfunktionellen Verbindungen mit einem Molekulargewicht von 62 bis 399 mol/g,

F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und

G) gegebenenfalls einer aliphatischen Polyisocyanat-Komponente mit einer mittleren Isocyanatfunktionalität von > 2,6 und ≤ 4,

wobei mindestens eine der Komponenten C2) oder D) in der Frisur-stabilisierenden Zusammensetzung enthalten ist, wobei die Polyurethanharnstoff-Dispersion Carboxygruppen oder Carboxylatgruppen aufweist sowie

(V2) eine Polycarbodiimid-Komponente.

Die Bestandteile, deren Verhältnisse sowie das Verfahren zu der Herstellung der Komponenten (V1) und (V2) entsprechen denen im Zusammenhang mit der erfindungsgemäßen Frisur-stabilisierenden Zusammensetzung beschriebenen Komponenten A) bis G). Die Komponenten (V1) und (V2) werden bevorzugt entsprechend dem erfindungsgemäßen Verfahren zur Herstellung der erfindungsgemäßen Frisur-stabilisierenden Zusammensetzung verarbeitet. Insbesondere bilden die beiden Komponenten (V1) und (V2) nach ihrem Mischen die erfindungsgemäße Zusammensetzung. Wie bereits oben beschrieben, können die beiden Komponenten (V1) und (V2) zumindest zum Teil vor, während oder nach dem Gestalten der Frisur gemischt werden. So können die Komponenten (V1) und (V2) gemischt werden und zu einem geeigneten Zeitpunkt auf das Haar aufgebracht werden, es kann aber auch eine der Komponenten (V1) oder (V2) vor der Gestaltung des Haares aufgebracht werden und die jeweils andere Komponente danach. Es ist auch denkbar, dass beide Komponenten (V1) und (V2) gleichzeitig oder nacheinander nach der Gestaltung der Frisur auf das Haar gebracht werden. Um einen besonders langanhaltenden Effekt der Frisur-stabilisierung durch die Frisur-stabilisierende Zusammensetzung zu erreichen, sollte in jedem Fall darauf geachtet werden, dass sich die beiden Komponenten (V1) und (V2) ausreichend berühren oder mischen können. Die beiden Komponenten (V1) und (V2) können als Kit in einem gemeinsamen Behältnis, das die beiden Komponenten jeweils in getrennten Behältnissen vorsieht, abgepackt sein oder völlig separat von einander in jeweils getrennten Behältnissen verpackt sein.

**Methoden:**

Sofern nicht abweichend gekennzeichnet, beziehen sich alle Prozentangaben auf das Gewicht und die Gesamtmenge bzw. auf das Gesamtgewicht der Zusammensetzungen.
Sofern nicht abweichend vermerkt, beziehen sich alle analytischen Messungen auf Messungen bei Temperaturen von 23 °C.
Die Feststoff- bzw. Festkörpergehalte wurden entsprechend DIN EN ISO 3251 durch Erhitzen einer ausgewogenen Probe auf 105 °C bis zur Gewichtskonstanz ermittelt. Bei Gewichtskonstanz wurde durch erneutes Auswiegen der Probe der Festkörpergehalt berechnet.
NCO-Werte wurden, wenn nicht ausdrücklich anders erwähnt, volumetrisch gemäß DIN-EN ISO 11909 bestimmt.
Die Kontrolle auf freie NCO-Gruppen wurde mittels IR-Spektroskopie (Bande bei 2260 $cm^{-1}$) durchgeführt.
Die angegebenen Viskositäten wurden mittels Rotationsviskosimetrie nach DIN 53019 bei 23 °C mit einem Rotationsviskosimeter der Firma Anton Paar Germany GmbH, Ostfildern, DE bestimmt (1 Pa s = 1 $N/m^2$*s).
Die Bestimmung der mittleren Teilchengrößen (angegeben ist das Zahlenmittel) der Polyurethan-Dispersionen erfolgte nach Verdünnen mit entionisiertem Wasser mittels Laserkorrelations-Spektroskopie (Gerät: Malvern Zetasizer 1000, Malver Inst. Limited).

Der pH-Wert wurde gemäß der in DIN ISO 976 beschriebenen Methode an der unverdünnten Probe gemessen.

## Experimente

Für die Esperimente aus Beispiel 1 und 2 wurden entweder Euro-Haarsträhnen gewellt (18 cm totale Länge, 8 cm Breite, 1,0 g +/- 0,2 Gewicht) benutzt oder glatte Euro-Haarsträhnen (19 cm totale Länge, 3 cm Breite, 1,0 g +/- 0,2 Gewicht). Die Länge der Strähnen entspricht L0.

### Vorbereitung bzw. Locken oder Glättung von Haarsträhnen (Schritt 0)):

Vor den Experimenten, wie in Beispielen 1 und 2 beschrieben, wurden die Haarsträhnen 1 Minute mit 0,3 g eines kommerziellen Silikonfreien Shampoo (der Firma Syoss "Volume Lift Shampoo") bei 38°5 C gewaschen. Die Haarsträhnen wurden anschließend 1 Minute bei 38°C ausgespült und mit einem herkömmlichen Kamm (breite Seite) durchgekämmt. Anschließend wurden die Haarsträhnen mit Hilfe eines Föhns 1 Minute bei ca.75°C getrocknet.

### Verwendete Substanzen

Die getesteten Substanzen werden mit folgenden Abkürzungen genannt:

| | |
|---|---|
| Produkt A: | Dispercoll U XP 2643, von Covestro Deutschland AG, wässerige Polyurethan Dispersion, Festgehalt 40%, (V1) |
| Produkt B: | Desmodur XP 2802, von Covestro Deutschland AG, Polycarbodiimide, Festgehalt 40%, (V2) |
| Produkt C: | Baycusan C1008, INCI: Polyurethane-48 von Covestro Deutschland AG, wässerige Dispersion, Festgehalt 30% |
| Produkt D: | Luviflex Soft (INCI: Acrylate copolymer), von BASF AG, Deutschland |
| Produkt E: | Amphomer (INCI: Ocylacrylamide/Acrylates/Butyllaminoethyl Methacrylate Copolymer), 100 % mit AMP (Amino Methylpropanol) auf pH 9,6 eingestellt, 2 Gew.-%, von Akzonobel |
| Produkt F: | Syntran PC 5100 (INCI: Polyacrylate 21 (and) acrylate/Dimethylaminoethyl Methacrylate Copolymer), commercially available from the supplier Interpolymer, wässrige Lösung, Festgehalt 25% |

## Lockenbildung:

Gestaltung einer Frisur: Formung der Strähnen zu Locken (Schritt 1)

Eine gewaschene Haarsträhne (Europäische glatte Haarsträhnen, vorbereitet wie im Schritt 0 beschrieben, wurde in einer Schale, die auf einer Waage stand gelegt. 1g einer 2 Gewicht-% (Festgehalt) wässerige Lösung wurde über die ganze Länge der Haarsträhne verteilt sodass die Lösung möglichst homogen verteilt war. Danach wurde die Haarsträhne gekämmt, bis sie komplett benetzt war. Dann wurde die Strähne mit 2 Fingern glatt gestrichen. Diese wurde dann auf einem Kaltwellwickler gedreht mit einem Abstand zwischen 2 und 3 mm. Das Aufdrehen wurde mit Hilfe eines Gewichts, das am Strähnenende befestigt war, im Uhrzeigersinn durchgeführt. Die Haarsträhne wurde mittels eines Gummis befestigt. Alle fertigen Strähnen wurden dann in die 50% Feuchtigkeitskammer gehängt. Die Strähnen wurden in der Feuchtigkeitskammer mindesten 18 Stunden getrocknet.

Nach dem Trocknen wurden die Strähnen an einer Seite gegen den Uhrzeigersinn abgewickelt. Es wurde die Länge der Strähnen als LS ermittelt.

### Beispiel 1: Lockenhaltevermögen in Feuchtigkeit (High Humidity Curl retention) von Produkt A und Produkt B (Polycabodiimide) allein und in Abmischungen

Behandlung in Feuchtigkeitskammer (Feuchtigkeits-Test): Die Locken wurden im Anschluss an die Vorbereitung aus Schritt 0, in eine 90% Feuchtigkeitskammer bei 30°C gehängt. Die Luftfeuchte wurde mit destilliertem Wasser eingestellt. Danach wurde das Lockenhaltevermögen mit einer Zentimeterskala, die festinstalliert in der Feuchtigkeitskammer hing nach festgelegten Zeitintervallen nach: 10 min, 20 min, 30 min, 45 min, 60 min, 2 h, 3 h, 4h, 5 h, 6 h und 24 h bestimmt. Anhand des 24 h Wertes wurde der LT Wert ermittelt.

L0 = Länge der Haarsträhne vor jeglicher Behandlung
LS = Länge der Haarsträhne nach Schritt 1 aber vor dem Feuchtigkeits-Test
LT = Länge der Locke nach Feuchtigkeits-Test (hier nach 24 Stunden bei 30°C und 90% Luftfeuchtigkeit)

$$\text{Lockenhaltenvermögen } in\ \% = 100 \text{ x } \frac{(\text{L0} - \text{LT})}{(\text{L0} - \text{LS})}$$

Tabelle 1: Ergebnisse mit verschiedenen erfindungsgemäßen $^{eg}$ und nicht-erfindungsgemäßen $^{neg}$ Mischungen behandelten Haaren und deren Lockenhaltevermögen bei hoher Luftfeuchtigkeit

| Beispiel | Produkt A : Produkt B [g:g] | Lockenhaltevermögen [%] |
|---|---|---|
| 1-a | 1 : 0 $^{neg}$ | 31 |
| 1-b | 0.5 : 0.5 $^{eg}$ | 73 |
| 1-c | 0.7 : 0.3 $^{eg}$ | 70 |
| 1-d | 0.9 : 0.1 $^{eg}$ | 47 |
| 1-e | 0 : 1 $^{neg}$ | < 30 |

In Tabelle 1 sind Beispiele erfindungsgemäßer Mischungen 1-b bis 1-d im Vergleich zu nicht-erfindungsgemäßen Mischungen der Komponenten (V1) in Form von Produkt A 1-a und (V2) in Form von Produkt B 1-e gezeigt. Es ist deutlich erkennbar, dass das nicht erfindungsgemäße Produkt A allein eine sehr niedrige Feuchtigkeitsbeständigkeit für zu Locken frisiertem Haar aufweist, wie das Beispiel 1-a zeigt. Das gleiche gilt für das nicht erfindungsgemäße Produkt B alleine in Beispiel 1.e. In beiden Beispielen, 1-a und 1-e wird lediglich ein Lockenhaltevermögen um oder weniger als 30 % erreicht. Durch Anwendung der erfindungsgemäßen Mischung aus den Komponenten (V1) als Produkt A und (V2) als Produkt B konnte die Wasserbeständigkeit deutlich erhöht werden, was sich in den prozentualen Lockenhaltevermögenszahlen von 47 bis 73 % für die Mischungen 1b bis 1-d zeigt. Darüber hinaus ist erkennbar, dass das Lockenhaltevermögen mit einer 1:1 Mischung 1-b aus Komponente (V1) und (V2) ein Maximum für die vermessenen Mischungen aufweist.

**Beispiel 2 : Lockenhaltevermögen in Wasser**

Behandlung in Wasser: Nach Vorbereitung wie in Schritt 0 beschrieben, wurden Europäische glatte Haarsträhnen mit 1g Lösung der Mischungen 2-a bis 2-c aufgetragen und wie in Schritt 1 beschrieben, im Haar verteilt. Anschließend wurde deren Lockenhaltevermögen in Wasser bestimmt.

2-a$^{eg}$: Produkt A : Produkt B im Verhältnis 1:1,2 Gew.-% Feststoffgehalt
2-b$^{neg}$: Produkt D : Produkt B im Verhältnis 1:1,2 Gew.-% Feststoffgehalt
2-c$^{neg}$: Produkt F : Produkt B im Verhältnis 1:1,2 Gew.-% Feststoffgehalt

Zur Bestimmung des Lockenhaltevermögens in Wasser wurde wie folgt vorgegangen: Eine Haarlocke wurde 30 Sekunden in ein Wasserbad bei 38°C getaucht. Dann wurde die Locke dem Wasserbad entnommen, nass aufgehängt und die Länge der Locke wurde unmittelbar gemessen (LT nass). Danach wurde die Locke bei Raumtemperatur vollständig luftgetrocknet und wieder deren Länge bestimmt (LT trocken).

L0 = Länge der Haarsträhne vor jeglicher Behandlung (= 19 cm)
LS = Länge der Haarsträhne nach Behandlung (gelockt) vor dem Test
LT nass = Länge der nassen Locke direkt nach 30 Sekunde im Wasser Bad
LT trocken = Länge der Locke nach 30 Sekunden im Wasser Bad und Trocknung bei Raumtemperatur

$$Wasserbeständigkeit\ (nass)\ in\ \% = 100 \text{ x } \frac{(\text{L0} - \text{LT nass})}{(\text{L0} - \text{LS})}$$

$$Wasserbeständigkeit\ (trocken)\ in\ \% = 100 \text{ x } \frac{(\text{L0} - \text{LT trocken})}{(\text{L0} - \text{LS})}$$

Tabelle 2: Ergebnisse von mit verschiedenen erfindungsgemäßen $^{eg}$ und nicht-erfindungsgemäßen $^{neg}$ Mischungen behandelten Haaren sowie deren Wasserbehandlung

| Beispiel | Lockenhaltenvermögen [%] | Wasserbeständigkeit nass [%] | Wasserbeständigkeit trocken [%] |
|---|---|---|---|
| 2-a$^{eg}$ | 76 | 58 | 42 |
| 2-b$^{neg}$ | 77 | 40 | 20 |
| 2-c $^{neg}$ | 77 | 36 | 27 |

Obwohl die drei Mischungen 2-a bis 2-c zum ähnlichen Lockenhaltenvermögen führen, erlaubt die Mischung 2-a (erfindungsgemäss) eine deutlich bessere Wasserbeständigkeit, sowohl mit nasse als auch trockene Haare gemessen.

**Beispiel 3: Wasserbeständigkeit und Shampoobeständigkeit von geglätteten Haaren**

Nach der Vorbereitung, wie in Schritt 0) beschrieben, wurden die nassen Haarsträhnen (Europäische gelockte Haarsträhnen (B0 = 4 bis 5 cm Breite) mit einer der Mischungen 3-a (erfindungsgemäß), 3-b, oder 3c (nicht-erfindungsgemäße Vergleichsbeispiele) behandelt. Danach wurden die Haare mit dem Föhn 3 Minuten bei 75°C getrocknet und 5 mal 3 Sekunde bei 230°C mit einem handelsüblichen Glätteisen geglättet und anschließend 12 Stunden bei 56 % rel.-Luftfeuchtigkeit bei 23 °C vollständig getrocknet worden.

3-a: Produkt A : Produkt B im Verhältnis 1:1, 2 Gew.-% Feststoffgehalt
3-b: Produkt C : Produkt B im Verhältnis 1:1, 2 Gew.-% Feststoffgehalt
3-c: Produkt F: Produkt B im Verhältnis 1:1, 2 Gew.-% Feststoffgehalt

Wasserbeständigkeit: Die Breite der jeweiligen geglätteten Strähne wurde zur Ermittlung von BG ermittelt. Danach wurde die Strähne 30 Sekunden in einem Wasserbad bei 38°C getaucht, dann wurde die Strähne aufgehängt und vollständig bei Raumtemperatur luftgetrocknet. Die Breite der Strähne wurde dann wieder zur Bestimmung von BW gemessen.

Tabelle 3: Ergebnisse von mit verschiedenen erfindungsgemäßen $^{eg}$ und nicht-erfindungsgemäßen $^{neg}$ Mischungen behandelten und geglätteten Haaren sowie deren Wasserbehandlung

| Mischung | Breite unbehandelt B0 [cm] | Breite geglättet BG [cm] | Breite BW nach 30 Sek im Wasser und Trocknung [cm] |
|---|---|---|---|
| 3-a$^{eg}$ | 4 | 2,5 | 2,0 |
| 3-b$^{neg}$ | 4 | 3 | 1 |
| 3-c$^{neg}$ | 5 | 2,5 | 5 |

Waschbeständigkeit: Die Breite der geglätteten Strähne wurde zur Ermittlung von BG gemessen. Danach wurde die Strähne 1 Minute bei 38°C mit einem kommerziellen Shampoo (Syoss Volume Lift) gewaschen. Dann wurden die Haare mit Wasser circa 30 Sekunden ausgespült. Anschließend wurde die Strähne aufgehängt und bei Raumtemperatur vollständig getrocknet. Schließlich wurde die Breite der Strähne zur Ermittlung von BST gemessen.

Wenn die Strähne noch glatt war (< 4 cm) wurde die gleiche Strähne erneut schamponiert und die Waschbeständigkeit wieder durch Ermittlung der Breite B2ST bestimmt.

Tabelle 3: Ergebnisse von mit verschiedenen erfindungsgemäßen $^{eg}$ und nicht-erfindungsgemäßen $^{neg}$ Mischungen behandelten und geglätteten Haaren sowie deren Shampoobehandlung

| Mischung | Breite geglättet BG [cm] | Breite nach 1 * Shampoo und Trocknung BST [cm] | Breite nach 2 * Shampoos und Trocknung B2ST[cm] |
|---|---|---|---|
| 3-a$^{eg}$ | 3 | 1,5 | 3 |
| 3-b$^{neg}$ | 2,5 | 0,5 | 1,5 |

(fortgesetzt)

| Mischung | Breite geglättet BG [cm] | Breite nach 1 * Shampoo und Trocknung BST [cm] | Breite nach 2 * Shampoos und Trocknung B2ST[cm] |
|---|---|---|---|
| 3-$c^{neg}$ | 3 | 5 | - |

Die Mischung 3-c zeigte weder Wasser- noch Shampoo-Beständigkeit. Die Mischung 2-b hat bessere Wasser- und Shampoo-Beständigkeit gezeigt, allerdings tendierten die Haare zum zusammenkleben, was zu einem nicht sehr ästhetischen Aussehen der Haare führte. Die Mischung 3-a (erfindungsgemäß) zeigt auf den geglätteten Haaren dagegen eine sehr gute Wasser- als auch Shampoo-Beständigkeit (selbst nach 2 Shampoo-Behandlungen) der Glättung der Haare, ohne dass die Haare dabei verklebten. Darüber hinaus fühlten sich die mit der Mischung 3-a behandelten Haare sehr sanft an (wie gesundes Haare) und sahen sehr natürlich aus.

**Patentansprüche**

1. Eine Frisur-stabilisierende Zusammensetzung beinhaltend mindestens die folgenden Komponenten:

   (V1) einen Polyurethanharnstoff, welcher erhältlich ist, durch Umsetzung von wenigstens

   A) einer Polyisocyanat-Komponente mit einer mittleren Isocyanatfunktionalität von $\geq 1{,}8$ und $\leq 2{,}6$,
   B) einer polymeren Polyol-Komponente,
   C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
   D) gegebenenfalls weiteren hydrophilierenden Komponenten, die unterschiedlich sind von C2),
   E) gegebenenfalls hydroxyfunktionellen Verbindungen mit einem Molekulargewicht von 62 bis 399 mol/g,
   F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
   G) gegebenenfalls einer aliphatischen Polyisocyanat-Komponente mit einer mittleren Isocyanatfunktionalität von $> 2{,}6$ und $\leq 4$,

   wobei der Polyurethanharnstoff mindestens eine der Komponenten C2) oder D) aufweist, und
   wobei der Polyurethanharnstoff Carboxygruppen oder Carboxylatgruppen aufweist
   (V2) eine Polycarbodiimid-Komponente.

2. Die Zusammensetzung nach Anspruch 1, wobei das Polyisocyanat ausgewählt ist aus der Gruppe bestehend aus 1,4-Butylendiisocyanat, 1,6-Hexamethylendiisocyanat (HDI), 2,2,4 und/oder 2,4,4-Trimethylhexamethylendiisocyanat, die isomeren Bis(4,4'-isocyanatocyclohexyl)methane oder deren Mischungen beliebigen Isomerengehalts, 1,4-Cyclohexylendiisocyanat, 1,4-Phenylendiisocyanat, 2,4- und/oder 2,6-Toluylendiisocyanat, 1,5-Naphthylendiisocyanat, 2,2'-und/oder 2,4'- und/oder 4,4'-Diphenylmethandiisocyanat, 1,3- und/oder 1,4-Bis-(2-isocyanato-prop-2-yl)-benzol (TMXDI), 1,3-Bis(isocyanatomethyl)benzol (XDI), Alkyl-2,6-diisocyanatohexanoat (Lysindiisocyanate) mit C1-C8-Alkylgruppen, sowie 4-Isocyanatomethyl-1,8-octandiisocyanat (Nonantriisocyanat) und Triphenylmethan-4,4',4''-triisocyanat oder einer Mischung aus mindestens zwei hiervon.

3. Die Zusammensetzung nach einem der vorhergehenden Ansprüche, wobei das Polyisocyanat der Polyisocyanat-Komponente A) ausgewählt ist aus einem aliphatischen oder cycloaliphatischen Polyisocyanat.

4. Die Zusammensetzung nach einem der vorhergehenden Ansprüche, wobei die Komponente B) Poly(propylenglykol)polyetherpolyole enthält oder daraus besteht.

5. Die Zusammensetzung nach einem der vorhergehenden Ansprüche, wobei die Komponente B) eine mittlere Molekularmasse in einem Bereich von 400 bis 4000 g/mol aufweist.

6. Die Zusammensetzung nach einem der vorhergehenden Ansprüche, wobei die Komponente B) ein Gemisch aus Poly(propylenglykol)polyetherpolyolen enthält oder daraus besteht, wobei sich die Poly(propylenglykol)polyetherpolyole in ihren zahlenmittleren Molekulargewichten um wenigstens 100 g/mol unterscheiden.

7. Die Zusammensetzung nach einem der vorhergehenden Ansprüche, wobei das Polyol B) ausgewählt ist aus der Gruppe bestehend aus Bernstein-, Methylbernstein-, Glutar-, Adipin-, Pimelin-, Kork-, Azelain-, Sebacin-, Nonandicarbon-, Decandicarbon-, Terephthal-, Isophthal-, o-Phthal-, Tetrahydrophthal-, Hexahydrophthal-, Cyclohexandicarbon-, Malein-, Fumar-, Malon- oder Trimellitsäure sowie Säureanhydride, wie o-Phthal-, Trimellit- oder Bernsteinsäureanhydrid oder deren Gemische mit mehrwertigen Alkoholen, wie z.B. Ethandiol, Di-, Tri-, Tetraethylenglykol, 1,2-Propandiol, Di-, Tri-, Tetrapropylenglykol, 1,3-Propandiol, Butandiol-1,4, Butandiol-1,3, Butandiol-2,3, Pentandiol-1,5, Hexandiol-1,6, 2,2-Dimethyl-1,3-propandiol, 1,4-Dihydroxycyclohexan, 1,4-Dimethylolcyclohexan, Octandiol-1,8, Decandiol-1,10, Dodecandiol-1,12 oder einer Mischung aus mindestens zwei hiervon.

8. Die Zusammensetzung nach einem der vorhergehenden Ansprüche, wobei die Komponente C) wenigstens eine aminofunktionelle Verbindung C1), die keine ionischen oder ionogenen Gruppen aufweist und eine aminofunktionelle Verbindung C2), die ionische oder ionogene Gruppen aufweist enthält.

9. Die Zusammensetzung nach einem der vorhergehenden Ansprüche, wobei es sich bei der Komponente D) ausschließlich um nichtionisch hydrophilierende Komponenten D1) handelt.

10. Die Zusammensetzung nach einem der vorhergehenden Ansprüche, wobei die Polycarbodiimid-Komponente (V2) aus aliphatischen oder cycloaliphatischen Polyisocyanaten hergestellt ist.

11. Die Zusammensetzung nach einem der vorhergehenden Ansprüche, wobei die Polycarbodiimid-Komponente (V2) Polycarbodiimid-Verbindungen, enthaltend mindestens drei Carbodiimidgruppen, entsprechend der Formel (I),

$$R^4\text{-}[R^1\text{-N=C=N}]_n\text{-}R^5 \qquad \text{(I)},$$

beinhaltet, wobei

n für eine ganze Zahl von 3 bis 100, bevorzugt von 3 bis 50, oder bevorzugt von 3 bis 20, oder bevorzugt von 3 bis 10 steht,
$R^1$ und $R^5$ jeweils unabhängig voneinander für lineare oder verzweigte, aliphatische oder cyclo-aliphatische, gegebenenfalls substituierte Gruppen mit 4 bis 100, oder bevorzugt von 4 bis 50, oder bevorzugt von 4 bis 20 C-Atomen steht.
$R^4$ für lineare oder verzweigte, aliphatische oder cyclo-aliphatische, gegebenenfalls substituierte Gruppen mit 1 bis 100, oder bevorzugt von 1 bis 50, oder bevorzugt von 1 bis 20 C-Atomen steht.

12. Verwendung mindestens eines Polyurethanharnstoffes (V1), gegebenenfalls zusammen mit einem Polycarbodiimid (V2), zur Herstellung einer Frisur-stabilisierenden Zusammensetzung die geeignet ist Frisuren wasserstabil zu generieren, wobei der Polyurethanharnstoff (V1) erhältlich ist durch Umsetzung von wenigstens

A) einer Polyisocyanat-Komponente mit einer mittleren Isocyanatfunktionalität von $\geq 1{,}8$ und $\leq 2{,}6$,
B) einer polymeren Polyol-Komponente,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionellen Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanatreaktiven Gruppen unter den gewählten Umsetzungsbedingungen mit den in der Reaktionsmischung vorhandenen Isocyanatgruppen reagiert und
G) gegebenenfalls einer aliphatischen Polyisocyanat-Komponente mit einer mittleren Isocyanatfunktionalität von $> 2{,}6$ und $\leq 4$,

wobei der Polyurethanharnstoff mindestens eine der Komponenten C2) oder D) aufweist, und wobei der Polyurethanharnstoff Carboxygruppen oder Carboxylatgruppen aufweist.

**13.** Eine Verwendung der Frisur-stabilisierenden Zusammensetzung gemäß einem der Ansprüche 1 bis 11, zur Verbesserung der Wasserbeständigkeit von Frisuren.

**14.** Ein Verfahren zur Gestaltung einer Frisur mit mindestens den Schritten:

F1) Behandeln des Haares eines Anwenders mit einer Frisur-stabilisierenden Zusammensetzung gemäß einem der Ansprüche 1 bis 11;
F2) gegebenenfalls Wärmebehandeln des Haares des Anwenders, wobei Schritt F2) vor und/oder nach Schritt F1) erfolgen kann;
F3) Formen des Haares zu einer Frisur;
F4) gegebenenfalls Trocknen des Haares;
F5) gegebenenfalls in Kontaktbringen des Haares mit Wasser;
F6) gegebenenfalls mindestens einmaliges Wiederholen mindestens eines Teils der Schritte F4) und F5.

**15.** Ein Verfahren zur Herstellung einer Frisur-stabilisierenden Zusammensetzung mindestens mit den Schritten:

i. Herstellen eines Polyurethanharnstoff (V1) aus einem isocyanatgruppenhaltigen Prepolymer a., welches erhältlich ist aus der Reaktion von mindestens den Komponenten

A) mindestens einem Polyisocyanat mit einer Funktionalität von $\geq 1{,}5$ bis $\leq 6$; mit
B) mindestens einem Polyol mit einer Funktionalität von $\geq 1{,}5$ bis $\leq 6$;

ii. Umsetzen des Prepolymer a. mit mindestens

C) einer Mischung C) aus primären und/oder sekundären aminofunktionelle Verbindung C1) und aus primären und/oder sekundären Diaminoverbindungen C2) zu dem Polyurethanharnstoff (V1);

iii. Mischen des Polyurethanharnstoffs (V1) mit mindestens einem Polycarbodiimid (V2) unter Erhalt der Frisur-stabilisierenden Zusammensetzung.

**16.** Ein Kit, aufweisend mindestens die Komponenten:

(V3) einen Polyurethanharnstoff, welcher erhältlich ist durch Umsetzung von wenigstens

A) einer Polyisocyanat-Komponente mit einer mittleren Isocyanatfunktionalität von $\geq 1{,}8$ und $\leq 2{,}6$,
B) einer polymeren Polyol-Komponente,
C) einer aminofunktionellen Kettenverlängerer-Komponente mit mindestens 2 isocyanatreaktiven Aminogruppen, enthaltend wenigstens eine aminofunktionelle Verbindung C1), die keine ionischen oder ionogenen Gruppen aufweist und/oder eine aminofunktionelle Verbindung C2), die ionische oder ionogene Gruppen aufweist,
D) gegebenenfalls weiteren hydrophilierenden Komponenten, die unterschiedlich sind von C2),
E) gegebenenfalls hydroxyfunktionellen Verbindungen mit einem Molekulargewicht von 62 bis 399 mol/g,
F) gegebenenfalls einer Verbindung, die genau eine isocyanatreaktive Gruppe aufweist, oder einer Verbindung, die mehr als eine isocyanatreaktive Gruppe aufweist, wobei nur eine der isocyanat reaktiven Gruppen unter den gewählten Umsetzungsbedingungen mit den in der Reaktionsmischung vorhandenen Isocyanatgruppen reagiert und
G) gegebenenfalls einer aliphatischen Polyisocyanat-Komponente mit einer mittleren Isocyanatfunktionalität von $> 2{,}6$ und $\leq 4$,

wobei mindestens eine der Komponenten C2) oder D) in dem Klebstoff enthalten ist,
wobei die Polyurethanharnstoff-Dispersion Carboxygruppen oder Carboxylatgruppen aufweist
sowie
(V4) eine Polycarbodiimid-Komponente.

Europäisches Patentamt
European Patent Office
Office européen des brevets

# EUROPÄISCHER RECHERCHENBERICHT

Nummer der Anmeldung
EP 18 15 8863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 02/08327 A1 (NOVEON IP HOLDINGS CORP [US]) 31. Januar 2002 (2002-01-31)<br>* Seite 28, Zeilen 1,2; Anspruch 32; Beispiele 1-3,17-19,25,26 *<br>----- | 1-16 | INV.<br>A61Q5/00<br>A61K8/87<br>C08L75/00<br>C08L79/00 |
| X | EP 2 028 205 A1 (BAYER MATERIALSCIENCE LLC [US]) 25. Februar 2009 (2009-02-25)<br>* Beispiele 1-5 *<br>----- | 12,13 | |
| X | US 6 613 314 B1 (ROLLAT ISABELLE [FR] ET AL) 2. September 2003 (2003-09-02)<br>* Anspruch 1; Beispiele 62-64 *<br>----- | 12,13 | |
| X | WO 2010/133660 A2 (OREAL [FR]; FEUILLETTE LAETITIA [FR])<br>25. November 2010 (2010-11-25)<br>* Seiten 47-49 *<br>----- | 12,13 | |
| X | WO 2010/133658 A2 (OREAL [FR]; FEUILLETTE LAETITIA [FR])<br>25. November 2010 (2010-11-25)<br>* Seiten 51-60 *<br>----- | 12,13 | **RECHERCHIERTE SACHGEBIETE (IPC)** |
| X | EP 1 970 391 A2 (BAYER MATERIALSCIENCE LLC [US]) 17. September 2008 (2008-09-17)<br>* Beispiel 7 *<br>----- | 12,13 | A61Q<br>A61K<br>C08L |
| X | DE 101 03 510 A1 (SEIKOH CHEMICAL CO [JP]) 23. August 2001 (2001-08-23)<br>* Beispiele 1-3,8,9 *<br>----- | 1-12,15, 16 | |
| X | WO 2012/068059 A1 (LUBRIZOL ADVANCED MAT INC [US]; SNOW GEORGE E [US])<br>24. Mai 2012 (2012-05-24)<br>* Beispiele 1,2,4,7,8 *<br>-----<br>-/-- | 1-12,15, 16 | |

Der vorliegende Recherchenbericht wurde für alle Patentansprüche erstellt

2

| Recherchenort | Abschlußdatum der Recherche | Prüfer |
|---|---|---|
| München | 30. Juli 2018 | Lanz, Sandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 1 von 3

Europäisches Patentamt
European Patent Office
Office européen des brevets

# EUROPÄISCHER RECHERCHENBERICHT

Nummer der Anmeldung
EP 18 15 8863

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 332 998 A1 (AIR PROD & CHEM [US]) 15. Juni 2011 (2011-06-15) * Beispiel 33; Tabelle V * ----- | 1-12,15, 16 | |
| X | DE 10 2009 049630 A1 (BAYER MATERIALSCIENCE AG [DE]; BENECKE KALIKO AG [DE]) 21. April 2011 (2011-04-21) * Absätze [0073], [0074], [0076], [0077] * ----- | 1-12,15, 16 | |
| X | DE 10 2010 015246 A1 (BAYER MATERIALSCIENCE AG [DE]; BENECKE KALIKO AG [DE]) 20. Oktober 2011 (2011-10-20) * Absätze [0079], [0080], [0082] * ----- | 1-12,15, 16 | |
| X | EP 3 101 172 A1 (TORAY INDUSTRIES [JP]) 7. Dezember 2016 (2016-12-07) * Absatz [0147]; Beispiel 3 * ----- | 1-12,15, 16 | |
| X | EP 3 211 132 A1 (TORAY INDUSTRIES [JP]) 30. August 2017 (2017-08-30) * Absätze [0215] - [0217] * ----- | 1-12,15, 16 | RECHERCHIERTE SACHGEBIETE (IPC) |
| X | EP 3 073 010 A1 (TORAY INDUSTRIES [JP]) 28. September 2016 (2016-09-28) * Absatz [0184] * ----- | 1-12,15, 16 | |
| X | US 6 235 384 B1 (VOSS PETER A [US] ET AL) 22. Mai 2001 (2001-05-22) * Beispiele 2,3 * ----- | 1-12,15, 16 | |
| X | EP 2 602 273 A1 (DAINIPPON INK & CHEMICALS [JP]) 12. Juni 2013 (2013-06-12) * Absätze [0105], [0106], [0123] * ----- | 1-12,15, 16 | |
| X | EP 1 591 502 A1 (BAYER MATERIALSCIENCE LLC [US]) 2. November 2005 (2005-11-02) * Beispiel 5 * ----- | 1-12,15, 16 | |
| | -/-- | | |

2

Der vorliegende Recherchenbericht wurde für alle Patentansprüche erstellt

| Recherchenort | Abschlußdatum der Recherche | Prüfer |
|---|---|---|
| München | 30. Juli 2018 | Lanz, Sandra |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 18 15 8863

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | WO 2014/010524 A1 (TATSUTA DENSEN KK [JP]) 16. Januar 2014 (2014-01-16)<br>* Beispiele 36,40 *<br>----- | 1-12,15, 16 | |
| | | | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 30. Juli 2018 | Lanz, Sandra |

2

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 18 15 8863

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-07-2018

| Im Recherchenbericht angeführtes Patentdokument | Datum der Veröffentlichung | Mitglied(er) der Patentfamilie | Datum der Veröffentlichung |
|---|---|---|---|
| WO 0208327 A1 | 31-01-2002 | AT 325837 T | 15-06-2006 |
| | | AU 2454902 A | 05-02-2002 |
| | | DE 60119526 T2 | 03-05-2007 |
| | | EP 1301564 A1 | 16-04-2003 |
| | | ES 2262693 T3 | 01-12-2006 |
| | | JP 5508655 B2 | 04-06-2014 |
| | | JP 2004504464 A | 12-02-2004 |
| | | KR 20030068534 A | 21-08-2003 |
| | | MX PA03000515 A | 14-10-2003 |
| | | TW I258489 B | 21-07-2006 |
| | | US 2002028875 A1 | 07-03-2002 |
| | | WO 0208327 A1 | 31-01-2002 |
| EP 2028205 A1 | 25-02-2009 | BR PI0803505 A2 | 12-05-2009 |
| | | CA 2637869 A1 | 08-02-2009 |
| | | CN 101361701 A | 11-02-2009 |
| | | EP 2028205 A1 | 25-02-2009 |
| | | JP 5410049 B2 | 05-02-2014 |
| | | JP 2009046673 A | 05-03-2009 |
| | | KR 20090015842 A | 12-02-2009 |
| | | TW 200914058 A | 01-04-2009 |
| | | US 7452525 B1 | 18-11-2008 |
| | | US 2009041689 A1 | 12-02-2009 |
| US 6613314 B1 | 02-09-2003 | AU 8238701 A | 13-02-2002 |
| | | EP 1307177 A2 | 07-05-2003 |
| | | JP 2004515466 A | 27-05-2004 |
| | | US 6613314 B1 | 02-09-2003 |
| | | US 2004141942 A1 | 22-07-2004 |
| | | WO 0209655 A2 | 07-02-2002 |
| WO 2010133660 A2 | 25-11-2010 | EP 2432448 A2 | 28-03-2012 |
| | | WO 2010133660 A2 | 25-11-2010 |
| WO 2010133658 A2 | 25-11-2010 | EP 2432449 A2 | 28-03-2012 |
| | | WO 2010133658 A2 | 25-11-2010 |
| EP 1970391 A2 | 17-09-2008 | AT 509970 T | 15-06-2011 |
| | | CA 2625135 A1 | 14-09-2008 |
| | | CN 101264048 A | 17-09-2008 |
| | | EP 1970391 A2 | 17-09-2008 |
| | | ES 2365180 T3 | 23-09-2011 |
| | | JP 5456982 B2 | 02-04-2014 |
| | | JP 2008231424 A | 02-10-2008 |
| | | US 2008226569 A1 | 18-09-2008 |
| | | US 2009022678 A1 | 22-01-2009 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 18 15 8863

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-07-2018

| Im Recherchenbericht angeführtes Patentdokument | Datum der Veröffentlichung | Mitglied(er) der Patentfamilie | Datum der Veröffentlichung |
|---|---|---|---|
| DE 10103510 A1 | 23-08-2001 | DE 10103510 A1 | 23-08-2001 |
| | | JP 4050438 B2 | 20-02-2008 |
| | | JP 2001207111 A | 31-07-2001 |
| | | US 2001011114 A1 | 02-08-2001 |
| WO 2012068059 A1 | 24-05-2012 | CN 103270066 A | 28-08-2013 |
| | | MY 161156 A | 14-04-2017 |
| | | TW 201225866 A | 01-07-2012 |
| | | US 2013225755 A1 | 29-08-2013 |
| | | WO 2012068059 A1 | 24-05-2012 |
| EP 2332998 A1 | 15-06-2011 | CN 102161743 A | 24-08-2011 |
| | | EP 2332998 A1 | 15-06-2011 |
| | | JP 5813947 B2 | 17-11-2015 |
| | | JP 2011140647 A | 21-07-2011 |
| | | JP 2015163714 A | 10-09-2015 |
| | | KR 20110068909 A | 22-06-2011 |
| | | TW 201125884 A | 01-08-2011 |
| | | US 2011306724 A1 | 15-12-2011 |
| DE 102009049630 A1 | 21-04-2011 | KEINE | |
| DE 102010015246 A1 | 20-10-2011 | KEINE | |
| EP 3101172 A1 | 07-12-2016 | CN 105940154 A | 14-09-2016 |
| | | EP 3101172 A1 | 07-12-2016 |
| | | JP WO2015115290 A1 | 23-03-2017 |
| | | KR 20160114069 A | 04-10-2016 |
| | | TW 201538627 A | 16-10-2015 |
| | | US 2017183814 A1 | 29-06-2017 |
| | | WO 2015115290 A1 | 06-08-2015 |
| EP 3211132 A1 | 30-08-2017 | CN 107002351 A | 01-08-2017 |
| | | EP 3211132 A1 | 30-08-2017 |
| | | JP WO2016063761 A1 | 03-08-2017 |
| | | KR 20170072893 A | 27-06-2017 |
| | | TW 201623732 A | 01-07-2016 |
| | | US 2017350069 A1 | 07-12-2017 |
| | | WO 2016063761 A1 | 28-04-2016 |
| EP 3073010 A1 | 28-09-2016 | CN 105745375 A | 06-07-2016 |
| | | EP 3073010 A1 | 28-09-2016 |
| | | JP WO2015076204 A1 | 16-03-2017 |
| | | KR 20160088330 A | 25-07-2016 |
| | | TW 201527344 A | 16-07-2015 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 18 15 8863

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-07-2018

| Im Recherchenbericht angeführtes Patentdokument | Datum der Veröffentlichung | Mitglied(er) der Patentfamilie | Datum der Veröffentlichung |
|---|---|---|---|
| | | US 2016289890 A1 | 06-10-2016 |
| | | WO 2015076204 A1 | 28-05-2015 |
| US 6235384 B1 | 22-05-2001 | CA 2239718 A1 | 04-12-1999 |
| | | US 6235384 B1 | 22-05-2001 |
| EP 2602273 A1 | 12-06-2013 | CN 103068873 A | 24-04-2013 |
| | | EP 2602273 A1 | 12-06-2013 |
| | | JP 5013233 B2 | 29-08-2012 |
| | | JP WO2012017724 A1 | 03-10-2013 |
| | | KR 20130098895 A | 05-09-2013 |
| | | TW 201211091 A | 16-03-2012 |
| | | US 2013136913 A1 | 30-05-2013 |
| | | WO 2012017724 A1 | 09-02-2012 |
| EP 1591502 A1 | 02-11-2005 | CA 2504957 A1 | 27-10-2005 |
| | | CN 1690146 A | 02-11-2005 |
| | | EP 1591502 A1 | 02-11-2005 |
| | | ES 2436751 T3 | 07-01-2014 |
| | | JP 2005314703 A | 10-11-2005 |
| | | KR 20060046699 A | 17-05-2006 |
| | | MX PA05004424 A | 23-11-2005 |
| | | SI 1591502 T1 | 31-03-2014 |
| | | US 2005238815 A1 | 27-10-2005 |
| | | US 2008242757 A1 | 02-10-2008 |
| WO 2014010524 A1 | 16-01-2014 | CN 104487534 A | 01-04-2015 |
| | | JP 5976112 B2 | 23-08-2016 |
| | | JP WO2014010524 A1 | 23-06-2016 |
| | | KR 20150035604 A | 06-04-2015 |
| | | TW 201418406 A | 16-05-2014 |
| | | WO 2014010524 A1 | 16-01-2014 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009118105 A1
- EP 934956 A
- US 3836537 A
- FR 1400366
- WO 2011120928 A2


### In der Beschreibung aufgeführte Nicht-Patentliteratur


- Ullmanns Encyclopädie der technischen Chemie. Verlag Chemie, vol. 19, 31-38
- International Cosmetic Ingredient Dictionary and Handbook. The Cosmetic, Toiletry, and Fragrance Association, 2002, vol. 4
- Microemulsions Theory and Practice. Academic Press, 1977, 21-32